# EUROPEAN PATENT SPECIFICATION

(11) **EP 4 299 083 B1**
(45) Date of publication and mention of the grant of the patent: **31.12.2025**
(21) Application number: 22781642.8
(22) Date of filing: 31.03.2022
(51) Int. Cl.: A61L 24/00, A61L 27/52, A61L 27/36, C12N 11/06, C12N 5/00, C12N 5/071, A61K 9/06

(54) **PHENOL DERIVATIVE-MODIFIED, TISSUE-DERIVED EXTRACELLULAR MATRIX DERIVATIVE FOR CONSTRUCTION OF ARTIFICIAL TISSUE**
PHENOLDERIVATMODIFIZIERTES, AUS GEWEBE GEWONNENES EXTRAZELLULÄRES MATRIXDERIVAT ZUR KONSTRUKTION VON KÜNSTLICHEM GEWEBE
DÉRIVÉ DE MATRICE EXTRACELLULAIRE DÉRIVÉ DE TISSU, MODIFIÉ PAR UN DÉRIVÉ DE PHÉNOL POUR LA CONSTRUCTION DE TISSU ARTIFICIEL

(30) Priority: 31.03.2021 KR 20210042240
(43) Date of publication of application: 03.01.2024
(73) Proprietor: Cellartgen Inc., Seoul 03722 (KR)
(72) Inventor: CHO, Seung Woo, Seoul 03722 (KR); AN, Soo Hwan, Namyangju-si Gyeonggi-do 12219 (KR); KIM, Su Kyeom, Seoul 04208 (KR); KIM, Yu Heun, Seoul 03726 (KR); KIM, Su Ran, Seoul 03725 (KR); MIN, Sung Jin, Seoul 03725 (KR); PARK, Se Won, Seoul 03722 (KR)
(74) Representative: Manitz Finsterwald Patent- und Rechtsanwaltspartnerschaft mbB
(86) International application number: PCT/KR2022/004595
(87) International publication number: WO 2022/211523

(56) References cited:
- JP-A- 2020 528 408
- CHO JUNG HO ET AL: "Ascidian-Inspired Fast-Forming Hydrogel System for Versatile Biomedical Applications: Pyrogallol Chemistry for Dual Modes of Crosslinking Mechanism", ADVANCED FUNCTIONAL MATERIALS, vol. 28, 15 December 2017 (2017-12-15), XP072408326, DOI: 10.1002/adfm.201705244
- SHIN MIKYUNG ET AL: "Gallol-derived ECM-mimetic adhesive bioinks exhibiting temporal shear-thinning and stabilization behavior", ACTA BIOMATERIALIA, ELSEVIER, AMSTERDAM, NL, vol. 95, 24 October 2018 (2018-10-24), pages 165 - 175, XP085782333, ISSN: 1742-7061, [retrieved on 20181024], DOI: 10.1016/J.ACTBIO.2018.10.028
- SHIN JISOO ET AL: "Tissue Reconstruction: Tissue Adhesive Catechol-Modified Hyaluronic Acid Hydrogel for Effective, Minimally Invasive Cell Therapy (Adv. Funct. Mater. 25/2015)", vol. 25, no. 25, 1 July 2015 (2015-07-01), DE, pages 3798 - 3798, XP055975016, ISSN: 1616-301X, Retrieved from the Internet <URL:https://api.wiley.com/onlinelibrary/tdm/v1/articles/10.1002%2Fadfm.201570167> DOI: 10.1002/adfm.201570167
- CHO JUNG HO ET AL: "Ascidian-Inspired Fast-Forming Hydrogel System for Versatile Biomedical Applications: Pyrogallol Chemistry for Dual Modes of Crosslinking Mechanism", ADVANCED FUNCTIONAL MATERIALS, vol. 28, 15 December 2017 (2017-12-15), XP055906558, DOI: 10.1002/adfm.201705244
- CHO JUNG HO, JUNG SEUNG LEE, JISOO SHIN, EUN JE JEON, SOOHWAN AN, YI SUN CHOI, SEUNG-WOO CHO: "Ascidian-Inspired Fast-Forming Hydrogel System for Versatile Biomedical Applications: Pyrogallol Chemistry for Dual Modes of Crosslinking Mechanism", ADVANCED FUNCTIONAL MATERIALS, vol. 28, 15 December 2017 (2017-12-15), XP055906558, DOI: 10.1002/adfm.201705244
- SHIN MIKYUNG; GALARRAGA JONATHAN H.; KWON MI Y.; LEE HAESHIN; BURDICK JASON A.: "Gallol-derived ECM-mimetic adhesive bioinks exhibiting temporal shear-thinning and stabilization behavior", ACTA BIOMATERIALIA, ELSEVIER, AMSTERDAM, NL, vol. 95, 24 October 2018 (2018-10-24), AMSTERDAM, NL, pages 165 - 175, XP085782333, ISSN: 1742-7061, DOI: 10.1016/j.actbio.2018.10.028
- WANG DANDAN; XU PINGPING; WANG SHUOSHUO; LI WENLI; LIU WEIZHI: "Rapidly curable hyaluronic acid-catechol hydrogels inspired by scallops as tissue adhesives for hemostasis and wound healing", EUROPEAN POLYMER JOURNAL, PERGAMON PRESS LTD OXFORD, GB, vol. 134, 25 May 2020 (2020-05-25), GB , XP086221049, ISSN: 0014-3057, DOI: 10.1016/j.eurpolymj.2020.109763
- SHIN JISOO, LEE JUNG SEUNG, LEE CHANGHYUN, PARK HYUN-JI, YANG KISUK, JIN YOONHEE, RYU JI HYUN, HONG KI SUNG, MOON SUNG-HWAN, CHUNG: "Tissue Reconstruction: Tissue Adhesive Catechol-Modified Hyaluronic Acid Hydrogel for Effective, Minimally Invasive Cell Therapy (Adv. Funct. Mater. 25/2015)", ADVANCED FUNCTIONAL MATERIALS, WILEY - V C H VERLAG GMBH & CO. KGAA, DE, vol. 25, no. 25, 1 July 2015 (2015-07-01), DE , pages 3798 - 3798, XP055975016, ISSN: 1616-301X, DOI: 10.1002/adfm.201570167

## Description

### TECHNICAL FIELD

The present disclosure relates to a phenol derivative-modified, tissue-derived extracellular matrix derivative for construction of artificial tissues.

### BACKGROUND

An extracellular matrix (ECM) refers to a three-dimensional network structure composed of macromolecules (collagen, elastin, glycoprotein, growth factor, etc.) that exist outside the cell but are closely related to the cell. The ECM is one of natural raw materials of hydrogel with excellent biocompatibility, biodegradability, strength, and flexibility.

An organoid that can be cultured in the ECM has recently attracted a great deal of interest because it is a three-dimensional cell structure composed of various cells constituting the tissue and thus can mimic the environment of living tissue. For this reason, it is widely used in various application fields, such as new drug development, disease modeling and regenerative therapy, as well as basic biology field.

Artificial tissues are man-made tissue and organ analogues that perform functions similar to human tissues and organs. Examples of the artificial tissues may include artificial joints, artificial bones, artificial skins, artificial tendons, artificial nerves, artificial tumors, etc. Also, examples of the artificial organs may include artificial livers, artificial kidneys, artificial hearts, artificial intestines, artificial skins, artificial blood vessels, etc. The artificial tissues need to have cell-proliferating potential and compartmentalization of plural types of cells in order to perform a function as a tissue rather than a simple cell aggregate, and also need to have the possibility of interactions between heterogeneous cells in order to mimic the tissue microenvironment. Therefore, a lot of research thereon has still been conducted.

In CHO JUNG HO ET AL: "Ascidian-Inspired Fast-Forming Hydrogel System for Versatile Biomedical Applications: Pyrogallol Chemistry for Dual Modes of Crosslinking Mechanism", ADVANCED FUNCTIONAL MATERIALS, vol. 28, 15 December 2017, DOI: 10.1002/adfm.201705244 a bioinspired hydrogel platform was developed, demonstrating versatile applicability for tissue engineering and drug delivery, by conjugating pyrogallol moiety ressembling ascidian 3,4,5-trihydroxyphenylalanine to hyaluronic acid.

SHIN MIKYUNG ET AL: "Gallol-derived ECM-mimetic adhesive bioinks exhibiting temporal shear-thinning and stabilization behavior", ACTA BIOMATERIALIA, ELSEVIER, AMSTERDAM, NL, vol. 95, 24 October 2018 , pages 165-175,ISSN: 1742-7061, DOI:10.1016/J.ACTBIO.2018.10.028 discloses a gallol-modified extracellular matrix (ECM) mimic obtained by gallol modification of ECM components (e.g., hyaluronic acid, gelatin).

In SHIN JISOO ET AL: "Tissue Reconstruction: Tissue Adhesive Catechol-Modified Hyaluronic Acid Hydrogel for Effective, Minimally Invasive Cell Therapy (Adv. Funct. Mater. 25/2015)",ADVANCED FUNCTIONAL MATERIALS, vol. 25, no. 25, 1 July 2015, pages 3798-3798, ISSN: 1616-301X, DOI: 10.1002/adfm.201570167 mussel-inspired, catechol-modified hyaluronic acid was shown to exhibit higher biocompatibility and improved tissue adhesiveness in comparison to HA hydrogel crosslinked via photopolymerization.

Accordingly, the present inventors conducted research on the construction of an organoid-based artificial tissue, and confirmed the possibility of fabricating an artificial tissue through organoid assembly using a phenol derivative-modified, tissue-derived extracellular matrix derivative. As a result, the present inventors achieved the present disclosure.

### DISCLOSURE OF THE INVENTION

### PROBLEMS TO BE SOLVED BY THE INVENTION

The present disclosure is conceived to provide a composition for hydrogel, comprising: a phenol derivative-modified, tissue-derived extracellular matrix.

The present disclosure is conceived to provide a hydrogel prepared by crosslinking the composition.

The present disclosure is conceived to provide a tissue adhesive composition comprising the hydrogel, a composition for cell culture comprising the hydrogel, a tissue structure to which the cell-coated composition adheres, a composition for drug delivery comprising the hydrogel, and a composition for cell transplantation comprising the hydrogel.

The present disclosure is conceived to provide a method of preparing a hydrogel, comprising: a process (a) of modifying a tissue-derived extracellular matrix with a phenol derivative; and a process (b) of crosslinking the modified, tissue-derived extracellular matrix to form a hydrogel.

### MEANS FOR SOLVING THE PROBLEMS

An aspect of the present disclosure provides a composition for hydrogel, comprising a phenol derivative-modified, tissue-derived extracellular matrix.

In an embodiment of the present disclosure, the phenol derivative may be derived from at least one of a catechol group ( 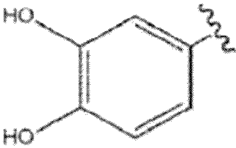 ) and a gallol group ( 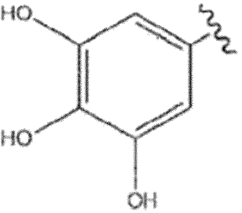 )

In an embodiment of the present disclosure, the tissue-derived extracellular matrix may be any one of a liver tissue-derived extracellular matrix, a lung tissue-derived extracellular matrix, a stomach tissue-derived extracellular matrix, an intestine tissue-derived extracellular matrix, a kidney tissue-derived extracellular matrix, a heart tissue-derived extracellular matrix, an esophageal tissue-derived extracellular matrix, a brain tissue-derived extracellular matrix, a spinal cord tissue-derived extracellular matrix, a pancreatic tissue-derived extracellular matrix, a uterine tissue-derived extracellular matrix, and an adipose tissue-derived extracellular matrix.

In an embodiment of the present disclosure, the weight ratio of the phenol derivative and the tissue-derived extracellular matrix may be 1:10 to 10:1.

Another aspect of the present disclosure provides a hydrogel prepared by crosslinking the composition.

In an embodiment of the present disclosure, the composition may have a concentration of 0.01%(w/v) to 3% (w/v).

In an embodiment of the present disclosure, the crosslinking may be oxidative crosslinking, and the oxidative crosslinking may be performed through at least one of a reaction by treatment with an oxidizing agent, a reaction under basic conditions, and natural oxidation.

In an embodiment of the present disclosure, the hydrogel may have adhesive, and the adhesiveness may be caused by at least one of a nucleophilic covalent bond, a hydrogen bond, a hydrophobic interaction, and a pi-pi interaction with the modified phenol derivative.

In an embodiment of the present disclosure, the hydrogel may be biodegradable.

Yet another aspect of the present disclosure provides a tissue adhesive composition including the hydrogel, a composition for cell culture including the hydrogel, a tissue structure to which the cell-coated composition adheres, a composition for drug delivery including the hydrogel, and a composition for cell transplantation including the hydrogel.

Still another aspect of the present disclosure provides a method of preparing a hydrogel, including: a process (a) of modifying a tissue-derived extracellular matrix with a phenol derivative; and a process (b) of crosslinking the modified, tissue-derived extracellular matrix to form a hydrogel.

In an embodiment of the present disclosure, the crosslinking in the process (b) may be oxidative crosslinking, and the oxidative crosslinking may be performed through at least one of a reaction by treatment with an oxidizing agent, a reaction under basic conditions, and natural oxidation.

In an embodiment of the present disclosure, the reaction by treatment with an oxidizing agent or the reaction under basic conditions may carried out by treating a substituted, tissue-derived extracellular matrix with at least one of NaOH, NalO₄, Na₂S₂O₈ and Fe³⁺.

### EFFECTS OF THE INVENTION

A hydrogel composition of the present disclosure and a hydrogel prepared therefrom exhibit various effects such as hemostasis, blood coagulation acceleration, cell transplantation, drug delivery, cell differentiation promotion, etc. and thus can be applied to single or composite uses. Further, the present disclosure is excellent in biocompatibility due to its biodegradability and being almost free of cytotoxicity, thus exhibiting very high applicability.

Furthermore, a tissue structure including a single type or multiple types of cells can be formed through the cell-proliferating potential and adhesion of the hydrogel of the present disclosure and can better simulate in-vivo microenvironments.

### BRIEF DESCRIPTION OF THE DRAWINGS

**FIG.** 1 is a schematic diagram showing the synthesis of an ECM-PG and the preparation of a hydrogel.
**FIG. 2** confirms the synthesis of the ECM-PG and shows the result of crosslinking.
**FIG.** 3 shows the result confirming assembly of ECM modules using the ECM-PG.
**FIG. 4** shows the result confirming assembly of an iPSC-derived liver organoid using an ECM-PG patch.
**FIG. 5A** shows the result of analysis of an iPSC-derived liver organoid structure prepared based on an ECM-PG patch. **FIG**. **5B** shows the result of analysis of an iPSC-derived liver organoid structure prepared based on an ECM-PG patch. **FIG. 5C** shows the result of analysis of an iPSC-derived liver organoid structure prepared based on an ECM-PG patch.
**FIG.** 6 shows the result of evaluation of toxicity of iPSC-derived liver organoids of ECM-PG hydrogels.
**FIG. 7A** shows the result of analysis of assembly of an iPSC-derived liver organoid using an ECM-PG hydrogel, and a mini-liver tissue structure. **FIG. 7B** shows the result of analysis of the assembly of an iPSC-derived liver organoid using an ECM-PG hydrogel, and a mini-liver tissue structure.
**FIG. 8A** shows the result of analysis of an iPSCs-derived mini-liver tissue structure prepared based on ECM-PG hydrogel. **FIG. 8B** shows the result of analysis of an iPSCs-derived mini-liver tissue structure prepared based on ECM-PG hydrogel. **FIG. 8C** shows the result of analysis of an iPSCs-derived mini-liver tissue structure prepared based on ECM-PG hydrogel.
**FIG. 9A** shows the result of preparation of an ECM-PG hydrogel-based iPSCs-derived mini-liver tissue structure containing liver microenvironment cells. **FIG. 9B** shows the result of preparation of an ECM-PG hydrogel-based iPSCs-derived mini-liver tissue structure containing liver microenvironment cells.
**FIG. 10A** shows the result of preparation of ECM-PG hydrogel-based mouse adult stem cell-derived liver tissue structure. **FIG. 10B** shows the result of preparation of ECM-PG hydrogel-based mouse adult stem cell-derived liver tissue structure.
**FIG. 11A** shows the result of assembly of an organoid (pancreatic organoid) using an ECM-PG hydrogel. **FIG. 11B** shows the result of assembly of an organoid (pancreatic organoid) using an ECM-PG hydrogel.
**FIG. 12A** shows the result of assembly of an organoid (lung organoid) using an ECM-PG hydrogel. **FIG. 12B** shows the result of assembly of an organoid (lung organoid) using an ECM-PG hydrogel.
**FIG. 13** shows the result of assembly of an organoid (kidney organoid) using an ECM-PG hydrogel.
**FIG. 14** shows the result of assembly of an organoid (intestinal organoid) using an ECM-PG hydrogel.
**FIG. 15** shows the result of assembly of an organoid (gastric organoid) using an ECM-PG hydrogel.
**FIG. 16A** shows the result of assembly of an organoid (esophageal organoid) using an ECM-PG hydrogel. **FIG. 16B** shows the result of assembly of an organoid (esophageal organoid) using an ECM-PG hydrogel.
**FIG. 17** shows the result of tissue adhesion test of ECM-PG patches.
**FIG. 18** shows the result of mass transplantation of an iPSC-derived liver organoid using ECM-PG patches.
**FIG. 19** shows the result of mass transplantation of an iPSC-derived liver organoid using ECM-PG patches.

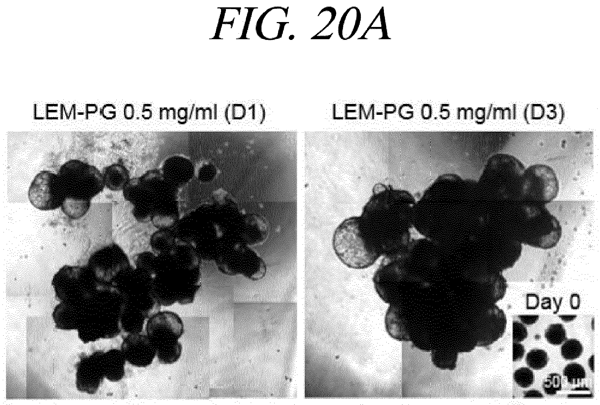

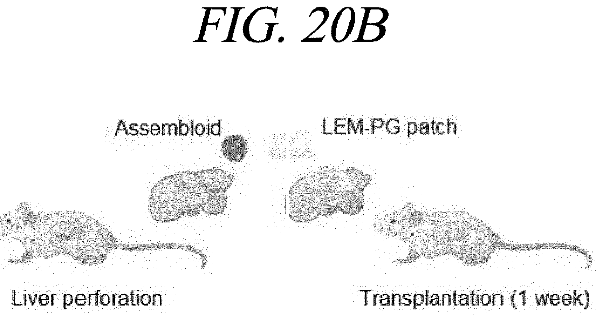

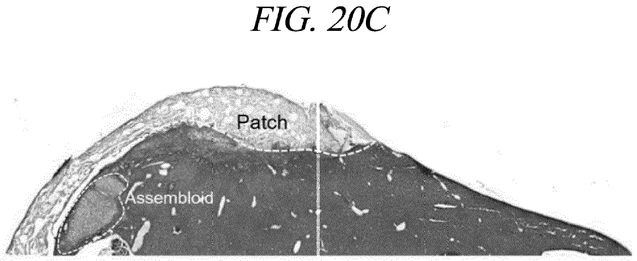

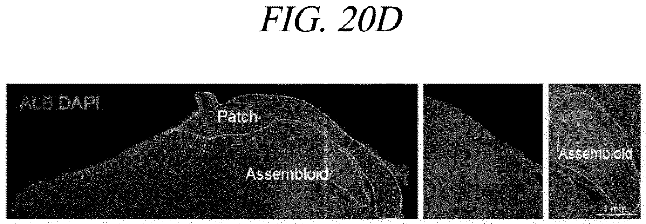

**FIG. 20A** shows the result of transplantation of an iPSC-derived mini-liver tissue structure prepared based on an ECM-PG hydrogel into a liver perforation model. **FIG. 20B** shows the result of transplantation of an iPSC-derived mini-liver tissue structure prepared based on an ECM-PG hydrogel into a liver perforation model. **FIG. 20C** shows the result of transplantation of an iPSC-derived mini-liver tissue structure prepared based on an ECM-PG hydrogel into a liver perforation model. **FIG. 20D** shows the result of transplantation of an iPSC-derived mini-liver tissue structure prepared based on an ECM-PG hydrogel into a liver perforation model.
**FIG. 21A** shows the result of verifying the effect of transplantation of an iPSC-derived mini-liver tissue structure prepared using an ECM-PG hydrogel into a partial liver resection model. **FIG. 21B** shows the result of verifying the effect of transplantation of an iPSC-derived mini-liver tissue structure prepared based on an ECM-PG hydrogel into a partial liver resection model.
**FIG. 22A** shows the result of verifying the effect of transplantation of an iPSC-derived mini-liver tissue structure prepared based on an ECM-PG hydrogel into a partial liver resection model. **FIG. 22B** shows the result of verifying the effect of transplantation of an iPSC-derived mini-liver tissue structure prepared based on an ECM-PG hydrogel into a partial liver resection model. **FIG. 22C** shows the result of verifying the effect of transplantation of an iPSC-derived mini-liver tissue structure prepared based on an ECM-PG hydrogel into a partial liver resection model.
**FIG. 23A** shows the result of verifying the effect of transplantation of an iPSC-derived mini-liver tissue structure prepared based on an ECM-PG hydrogel into a partial liver resection model. **FIG. 23B** shows the result of verifying the effect of transplantation of an iPSC-derived mini-liver tissue structure prepared based on an ECM-PG hydrogel into a partial liver resection model

### BEST MODE FOR CARRYING OUT THE INVENTION

An aspect of the present disclosure provides a composition for hydrogel, including a phenol derivative-modified, tissue-derived extracellular matrix.

In an embodiment of the present disclosure, the phenol derivative may be derived from at least one of a catechol group ( 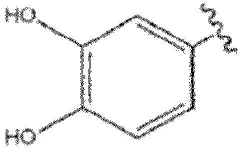 ) and a gallol group ( 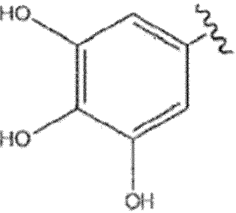 ).

In the present disclosure, the term "catechol group" refers to a substituent containing catechol, and the term "gallol group" refers to a substituent containing gallol. As a result of study on the polymerization through oxidation of the catechol group and the gallol group, they are known to be used as adhesive functional groups.

The tissue-derived extracellular matrix is a collection of biopolymers that fill the space inside or outside the tissue and is known as including fibrous proteins such as collagen and elastin, complex proteins such as proteoglycan and glycosaminoglycan, cell adhesion proteins such as fibronectin, laminin, vitronectin, etc. Meanwhile, in the present disclosure, the tissue-derived extracellular matrix can be obtained through decellularization techniques widely known in the art, such as a decellularization solution, and the tissue can include all biological tissues containing an extracellular matrix component. However, in the composition for hydrogel, the tissue may be any one or more of a liver tissue-derived extracellular matrix, a lung tissue-derived extracellular matrix, a stomach tissue-derived extracellular matrix, an intestine tissue-derived extracellular matrix, a kidney tissue-derived extracellular matrix, a heart tissue-derived extracellular matrix, an esophageal tissue-derived extracellular matrix, a brain tissue-derived extracellular matrix, a spinal cord tissue-derived extracellular matrix, a pancreatic tissue-derived extracellular matrix, a uterine tissue-derived extracellular matrix, and an adipose tissue-derived extracellular matrix.

As will be described later, the composition may become a hydrogel through crosslinking, specifically, oxidative crosslinking.

In the present disclosure, the term "hydrogel" is a gel containing water as a dispersion medium or basic ingredient. In the present disclosure, the hydrogel includes a tissue-derived extracellular matrix modified with a phenol derivative.

In an embodiment of the present disclosure, the composition may include a structure represented by the following Chemical Formula 1:

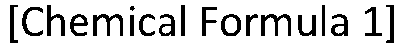

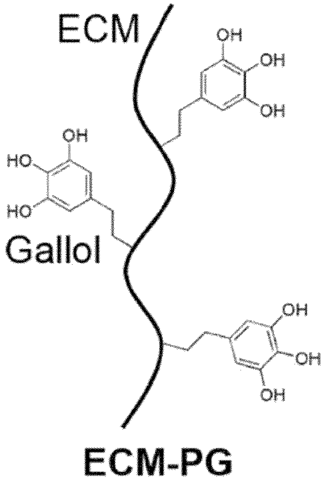

In an embodiment of the present disclosure, the weight ratio of the phenol derivative and the tissue-derived extracellular matrix may be 1:10 to 10:1, specifically 1:8 to 8:1, and most specifically 5:1. However, if it is out of the range, the desired effect of the present disclosure cannot be obtained.

Another aspect of the present disclosure provides a hydrogel prepared by crosslinking the composition.

In an embodiment of the present disclosure, when the hydrogel is prepared, the composition may have a concentration of 0.01% (w/v) to 3% (w/v), specifically 1% (w/v) to 3% (w/v), and more specifically 2% (w/v).

The hydrogel may be prepared by (a) modifying a tissue-derived extracellular matrix with a phenol derivative, and (b) crosslinking the modified, tissue-derived extracellular matrix to form a hydrogel. The crosslinking may include chemical crosslinking caused by UV irradiation, physical crosslinking, or biological crosslinking. Here, the chemical crosslinking by UV irradiation includes photo-crosslinking or crosslinking utilizing a reactive crosslinker. Also, the biological crosslinking includes crosslinking utilizing a binding force between heparin and growth factor or crosslinking using complementary bonding of DNA. Further, the physical crosslinking includes crosslinking by hydrogen bonding, crosslinking by hydrophobic interaction or crosslinking utilizing electrostatic interaction. Desirably, the crosslinking may be performed using an oxidizing agent or pH adjuster.

In an embodiment of the present disclosure, the crosslinking may be oxidative crosslinking, and more specifically, the oxidative crosslinking may be performed through at least one of a reaction by treatment with an oxidizing agent, a reaction under basic conditions, and natural oxidation.

The hydrogel may be a compound represented by the following Chemical Formula 2 or 3 and formed as a result of crosslinking. Specifically, a hydrogel may be formed of a compound of Chemical Formula 2 as a result of crosslinking with NalO₄ or a compound of Chemical Formula 3 as a result of crosslinking with NaOH.

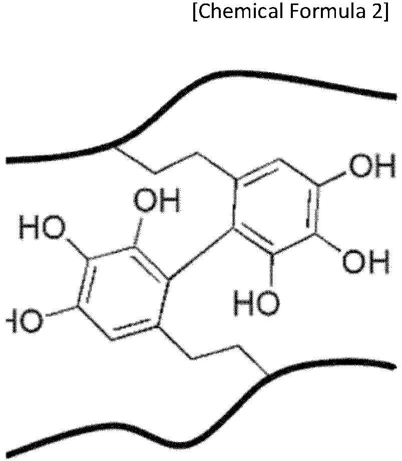

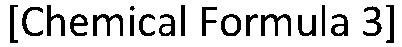

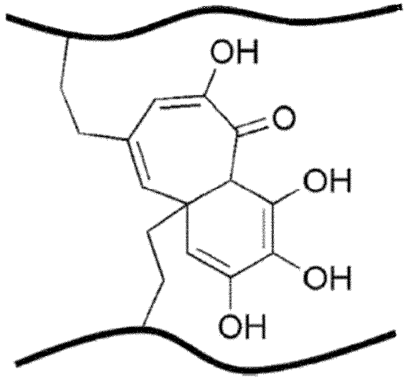

In an embodiment of the present disclosure, the hydrogel may exhibit one or more uses selected from the group consisting of blood coagulation acceleration, hemostasis, cell differentiation promotion, cell transplantation, and drug delivery. The hydrogel may have adhesiveness, and specifically, the adhesiveness may be caused by at least one of a nucleophilic covalent bond, a hydrogen bond, a hydrophobic interaction, and a pi-pi interaction with the modified phenol derivative. More specifically, the adhesiveness may be caused by at least one of a nucleophilic covalent bond, a hydrogen bond, a hydrophobic interaction, and a pi-pi interaction between the modified phenol derivative and various functional groups present on the cell surface and drug.

Also, the hydrogel may be biodegradable.

Yet another aspect of the present disclosure provides a tissue adhesive composition including the hydrogel. The hydrogel exhibits adhesiveness as described above, and can be used for adhesion between tissues.

Still another aspect of the present disclosure provides a composition for cell culture including the hydrogel. Specifically, the culture may be a three-dimensional culture, and the cells to be cultured are stem cells, hematopoietic stem cells, hepatocytes, fibroblasts, epithelial cells, mesothelial cells, endothelial cells, muscle cells, nerve cells, immune cells, adipocytes, chondrocytes, osteocytes, blood cells, or skin cells, but are not limited thereto. The culture is applicable to all cells that can be grown in the hydrogel of the present disclosure regardless of cell type. More specifically, the culture may be a co-culture of two or more types of cells.

Also, the present disclosure provides a tissue structure to which the cell-coated composition adheres. When cells are coated onto the composition, specifically, the hydrogel, and the cells are cultured, the cell-coated hydrogel adheres to another cell-coated hydrogel to form a tissue structure. This is due to the fact that the above-described hydrogel of the present disclosure has adhesiveness and can culture cells. Instead of simply culturing an organoid in the hydrogel, an environment of a tissue structure or organ can be more specifically mimicked by culturing one or plural types of cells together.

Still another aspect of the present disclosure provides a composition for drug delivery including the hydrogel.

In an embodiment of the present disclosure, the drug may be included in the hydrogel.

In an embodiment of the present disclosure, the drug may be selected from the group consisting of an immune cell activator, an anticancer agent, a therapeutic antibody, an antibiotic, an antibacterial agent, an antiviral agent, an anti-inflammatory agent, a contrast medium, a protein drug, a growth factor, a cytokine, a peptide drug, a hair growth solution, an anesthetic and combinations thereof.

Still another aspect of the present disclosure provides a composition for cell transplantation including the hydrogel. In an embodiment of the present disclosure, all types of cells for the purpose of transplantation may be used as the cells, and the location and method of transplantation of the composition are the same as those known in the art.

Still another aspect of the present disclosure provides a method of preparing a hydrogel, including: a process (a) of modifying a tissue-derived extracellular matrix with a phenol derivative; and a process (b) of crosslinking the modified, tissue-derived extracellular matrix to form a hydrogel.

The process (a) is a process of modifying a tissue-derived extracellular matrix with a phenol derivative. In an embodiment, a carboxyl group (-COOH) of the tissue-derived extracellular matrix may be bonded to an amine group of the phenol derivative, or an amine group of the tissue-derived extracellular matrix may be bonded to a carboxy group of the phenol derivative. In a most specific embodiment, a compound represented by Chemical Formula 1 may be prepared:

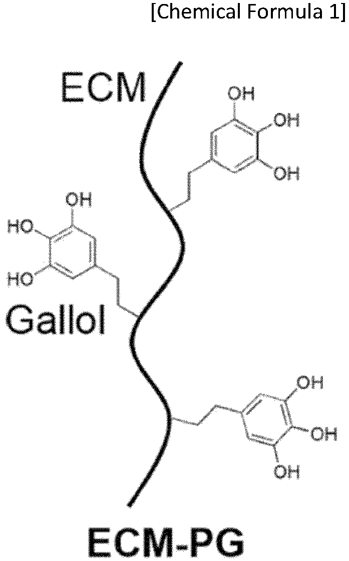

The phenol derivative may be any one or more of a catechol group ( 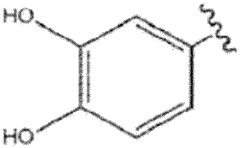 ) and a gallol group ( 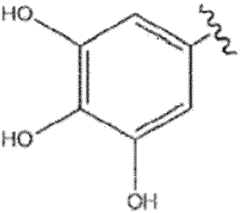 ). Specifically, by substituting the tissue-derived extracellular matrix with the phenol derivative, the tissue-derived extracellular matrix and the phenol derivative can be mixed and then treated with EDC/NHS at room temperature for 24 hours. The substitution may be performed by specifically mixing the tissue-derived extracellular matrix with EDC/NHS, stirring at a pH of 4 to 8, more specifically at a pH of 5.0 to 6.0, adding the phenol derivative, adjusting a pH to a range between 3.8 and 6.0, e.g., between 4.5 to 5.0 in the case where the phenol derivative is dopamine or between 3.8 and 5.0 in the case where the phenol derivative is 5-hydroxydopamine, and then stirring (EDC/NHS chemistry).

The process (b) is a process of crosslinking the modified, tissue-derived extracellular matrix to form a hydrogel. The crosslinking in the process (b) may be oxidative crosslinking, and more specifically, the crosslinking may be performed through at least one of a reaction by treatment with an oxidizing agent, a reaction under basic conditions, and natural oxidation. The reaction by treatment with an oxidizing agent or the reaction under basic conditions may carried out by treating a substituted, tissue-derived extracellular matrix with at least one of NaOH, NaIO₄, Na₂S₂O₈ and Fe³⁺.

Specifically, phenol derivatives of the modified, tissue-derived extracellular matrix may be crosslinked, and as a result of crosslinking between the phenol derivatives, quinone, semi-quinone intermediate, phenoxy radical, etc. may be formed.

### MODE FOR CARRYING OUT THE INVENTION

Hereinafter, one or more specific embodiments will be described in more detail through examples. However, these examples are for illustrative purposes of one or more embodiments, and the scope of the present disclosure is not limited to these examples.

### Example 1: Synthesis of ECM-PG, and preparation and characterization of hydrogel

### Example 1-1. Synthesis of ECM-PG and preparation of hydrogel

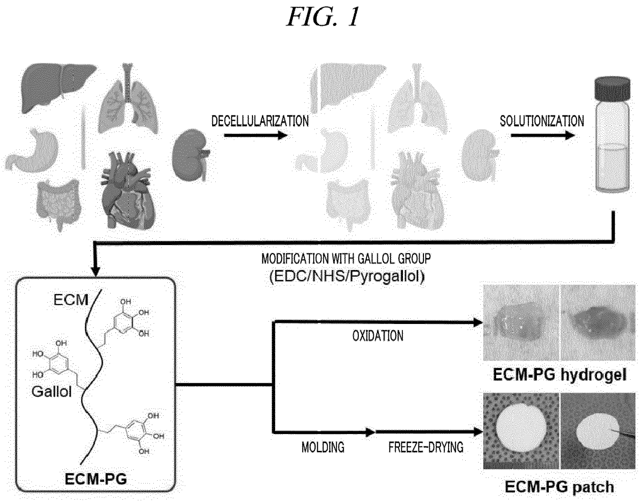

An extracellular matrix (ECM) was obtained by subjecting a living tissue to appropriate decellularization and solutionization, and then modified with pyrogallol (PG) through a chemical reaction (EDC/NHS chemistry) to synthesize a gallol-modified, tissue-derived extracellular matrix (ECM-PG) **(****FIG. 1****).**

Specifically, a reactant with a mass ratio of ECM:5-hydroxydopamine HCl of 1:5 and a molar ratio of 5-hydroxydopamine HCL:EDC:NHS of 1:1:2 was allowed to react in a 0.05 M 2-(N-morpholino)ethanesulfonic acid (MES) solution at room temperature for 6 hours, followed by dialysis using acidic PBS and water to remove the unreacted material and synthesize ECM-PG. In the synthesized ECM-PG solution (The ECM-PG material was prepared by dissolving the final 1X PBS in a neutral buffer. The final material concentration can be variously adjusted depending on the use.), crosslinking between polymers can be induced through oxidation (treatment with an oxidizing agent or natural oxidation), and, thus, a hydrogel can be formed. Also, various types of ECM-PG patches can also be obtained by molding and freeze-drying (until the frozen moisture was completely removed) the ECM-PG solution to a desired size and shape.

### Example 1-2. Confirmation of synthesis of ECM-PG and crosslinking

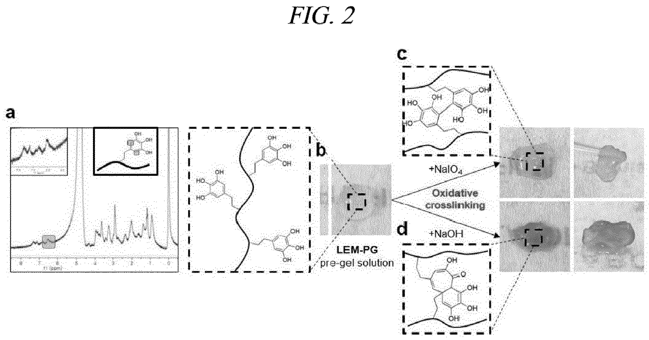

An ECM derivative, which was synthesized through the process described above, was analyzed. Herein, for example, a liver extracellular matrix (LEM), which is an ECM derived from liver tissue, was used. It was confirmed through a ¹H-NMR analysis that the ECM component of the synthesized ECM-PG was well modified with the gallol group. A phenyl proton (blue box) present in the ring structure of the gallol group was observed in the ECM-PG sample (please see a reference character "a" in FIG. 2).

It was confirmed that when the synthesized ECM-PG derivative was dissolved in a PBS buffer to prepare a pre-gel solution (please see a reference character "b" in **FIG. 2****)** and crosslinking was induced, the crosslinking was conducted in different ways depending on the oxidizing agent treatment (NalO₄) and basic pH (+NaOH) as in a conventionally reported gallol-modified polymer derivative, which resulted in the formation of a hydrogel (final hydrogel concentration: 2% (w/v), final NalO₄ concentration for crosslinking with NalO₄: 1.125 mg/ml, and final NaOH concentration for crosslinking with NaOH: 0.020 M) (please see a reference character "c" and "d" in FIG. 2)

### Example 2. Confirmation of possibility of assembly

### Example 2-1. Assembly of ECM modules using the ECM-PG

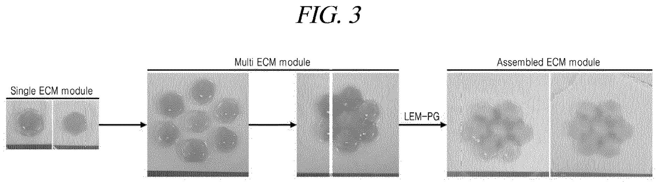

The strong reactivity of the gallol group of the ECM-PG to the nucleophilic functional group enables strong adhesion of the ECM-PG to an ECM hydrogel module composed of ECM proteins. Thus, it is possible to induce efficient assembly of ECM modules. The LEM-PG hydrogel of Example 1-2 was coated between the modules while inducing crosslinking by treatment with NaOH, and then allowed to react for 30 minutes to induce assembly (final LEM-PG hydrogel concentration: 2% (w /v) and final NaOH concentration: 0.02 M). In the case of three-dimensional cell culture including organoids, most cells grow within the ECM. Therefore, it was confirmed that when assembly of ECM unit modules (LEM) containing cells is induced to fabricate a large-sized organ-level tissue structure, the ECM-PG (LEM-PG) can serve as an adhesive **(****FIG. 3****).**

### Example 2-2. Assembly of iPSC-derived liver organoid using ECM-PG patch

An attempt was made to fabricate an artificial tissue through organoid assembly using the ECM-PG. As an example, the LEM-PG was used to induce assembly of liver organoids.

The liver organoids were prepared by differentiating human induced pluripotent stem cells (hiPSCs) into hepatic endoderm cells and mixing human umbilical vein endothelial cells (HUVECs) with human mesenchymal stem cells (hMSCs). The liver organoids were formed by mixing hiPSC-derived hepatocytes, HUVECs and hMSCs at a ratio of 10:7:2.

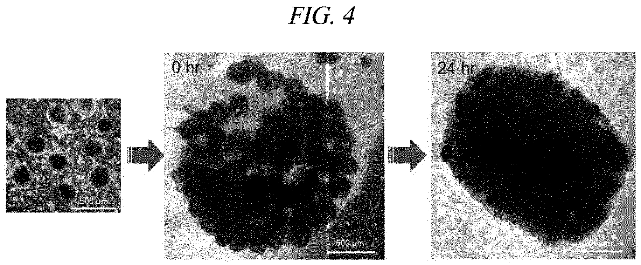

In consideration of the size of the tissue structure to be prepared, 50 to 100 or more liver organoids (herein, 100 liver organoids) were collected and coated onto a gallol-modified, liver tissue-derived extracellular matrix (LEM-PG) patch. After the patch was rolled up and cultured for 30 minutes with the organoids covered, crosslinking between LEM-PG derivatives was spontaneously promoted through natural oxidation, which made it possible to stably form a single structure. Thereafter, it was confirmed that the liver organoids coated onto the adhesive patch were incorporated into a large structure within 24 hours through dynamic culture in a shaker, which made it possible to prepare a structure at the liver tissue level. It was confirmed that a single liver organoid had a size of about 200 µm to about 300 µm, whereas a liver tissue prepared through assembly of 100 organoids was formed to a size of about 3 mm **(****FIG. 4****).**

### Example 3: Analysis of iPSC-derived liver organoid prepared using ECM-PG patch

### Example 3-1. Analysis of iPSC-derived liver organoid structure prepared using ECM-PG patch

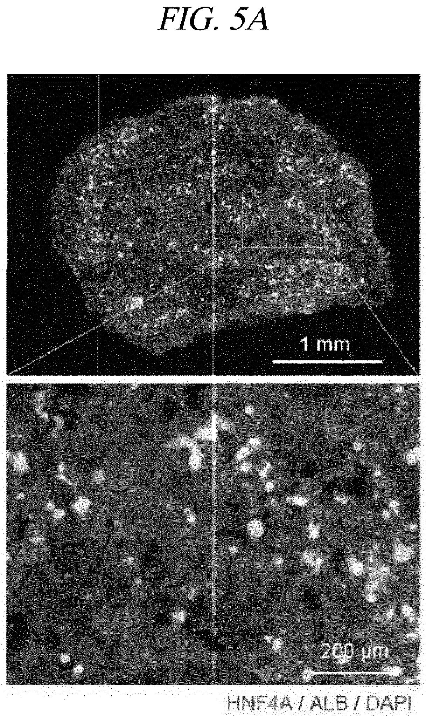

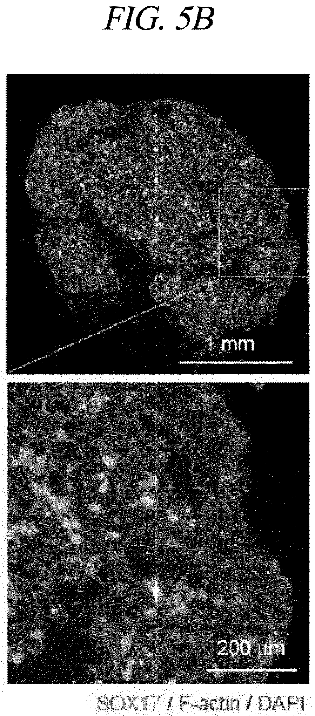

To analyze a differentiation marker in a liver tissue formed through assembly of 100 iPSC-derived liver organoids using an LEM-PG patch (after the patch coated with organoids was rolled up and cultured for 30 minutes with the organoids covered, crosslinking between LEM-PG derivatives was spontaneously promoted through natural oxidation and a single structure was stably formed), immunostaining was performed after culture for 48 hours. It was confirmed that hepatic differentiation markers (HNF4A and ALB), a hepatic endoderm marker (SOX17), and an actin filament (F-actin) were well expressed in the liver tissue with a size of about 3 mm. **(****FIGS. 5A** and **5B****).**

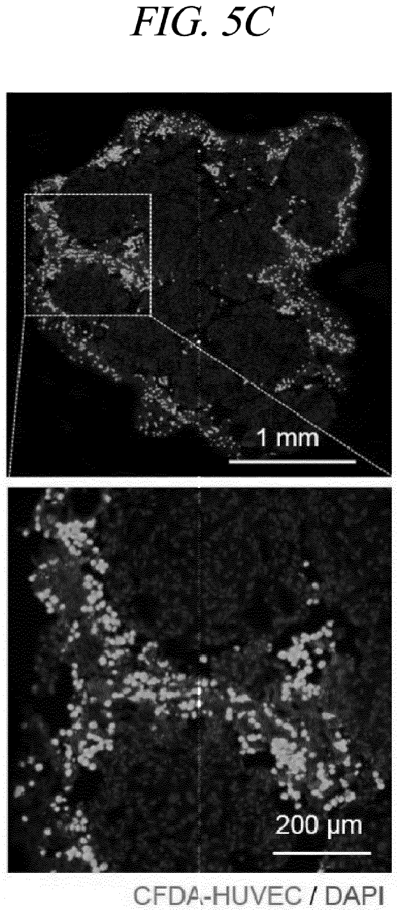

Further, when organoids were coated onto the gallol-modified liver tissue-derived extracellular matrix (LEM-PG) patch, vascular endothelial cells labelled with CFDA fluorescent reagent were also coated to induce the formation of a liver tissue. Then, when immunostaining was performed, vascular cells were evenly distributed between the organoid structures **(****FIG. 5C****),** which shows a liver tissue including a vascular structure can be prepared when liver organoids are conjugated using the LEM-PG.

### Example 3-2. Evaluation of toxicity of iPSC-derived liver organoid of ECM-PG hydrogel

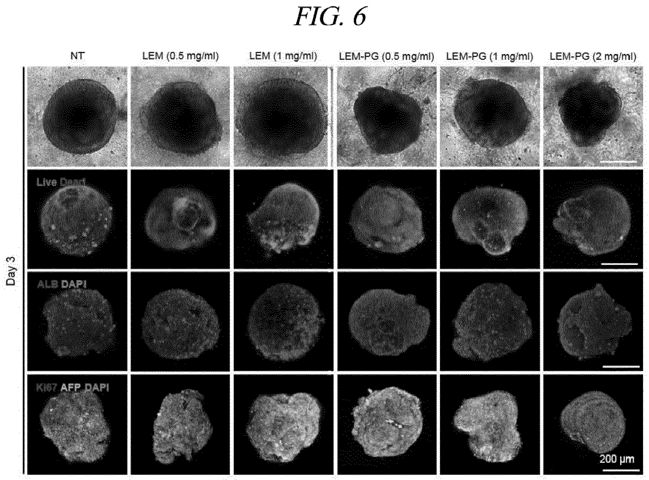

Before a mini-liver structure was prepared through assembly by coating the prepared ECM-PG onto a unit organoid in the form of a hydrogel, the hydrogel was added at different concentrations to the culture medium and the cell viability and expression of hepatic differentiation marker proteins were analyzed to check whether the ECM-PG hydrogel is toxic to the organoid. A pre-gel solution of the LEM-PG hydrogel was added at different concentrations to the hiPSC-derived liver organoid culture medium (the final concentration of the material in the culture medium is shown in **FIG. 6****),** and 3 days later, an analysis was performed.

When Live/Dead staining analysis was performed to analyze the viability of liver organoids, apoptosis was hardly observed in the group added with a decellularized liver tissue-derived hydrogel (LEM) and the groups added with the LEM-PG hydrogel at different concentrations, as in the group (No treatment; NT) where no hydrogel was added to the culture medium. It was confirmed that hepatic differentiation markers ALB and AFP were well expressed in the groups where LEM-PG was added, as in the NT group and the group where only the LEM was added. The expression level of Ki67, a cell proliferation marker, was maintained high in the NT group and the LEM group, but the expression level was relatively low in the LEM-PG groups. Therefore, it was confirmed that differentiation rather than proliferation occurs more in the organoid added with the LEM-PG **(****FIG. 6****).**

The gallol-modified LEM-PG hydrogel was confirmed as a non-toxic material suitable for liver organoid culture. The concentration of the LEM-PG group (0.5 mg/ml) with the highest viability and expression levels of differentiation markers was selected as the most suitable concentration for organoid assembly and applied to subsequent tests.

### Example 4: Assembly and analysis of iPSC-derived liver organoid using ECM-PG hydrogel

### Example 4-1. Assembly of iPSC-derived liver organoid using ECM-PG hydrogel and analysis of mini-liver tissue construct

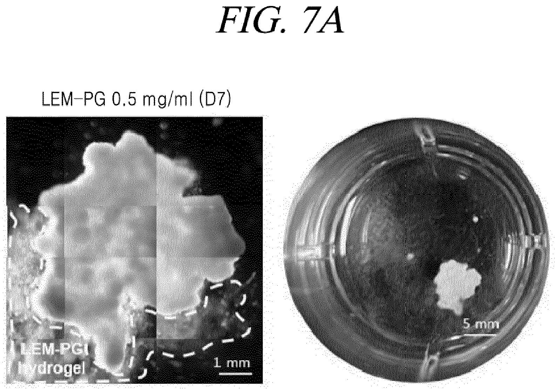

The optimized 5 mg/ml LEM-PG hydrogel was coated onto 200 hiPSC-derived liver organoids at a concentration of 10% (v/v) (final concentration in the culture medium of the LEM-PG: 0.5 mg/ml) to induce assembly of the liver organoids. Through culture in a shaker after coating with the LEM-PG hydrogel, the organoids were conjugated within 3 days and a mini-liver structure with a size of 5 mm to 6 mm was formed **(****FIG. 7A****).** Then, after maturation through additional culture for a total of 7 days, immunostaining was performed to analyze the mini-liver structure.

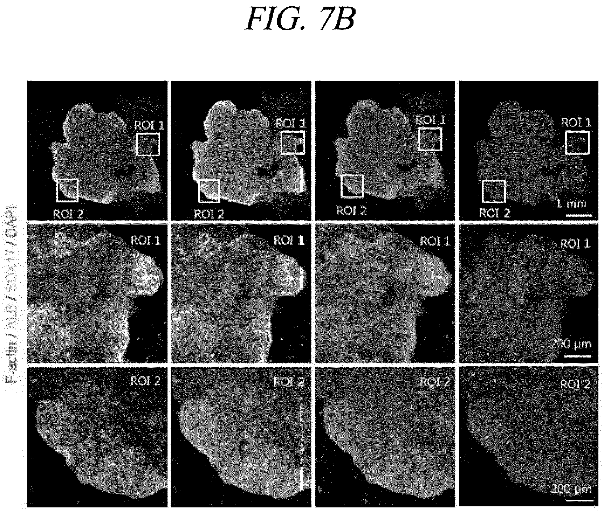

It was confirmed that when the mini-liver structure cultured for 7 days was stained for hepatic differentiation markers ALB and SOX17, both SOX17, both an early endoderm differentiation marker, and ALB, a mature hepatic differentiation marker, were well expressed in the organoid inside the structure **(****FIG. 7B****).** It was also confirmed that assembly of unit organoids was well made inside through staining for F-actin, a fibrous filament marker **(****FIG. 7B****).**

### Example 4-2. Analysis of iPSC-derived mini-liver tissue prepared based on ECM-PG hydrogel

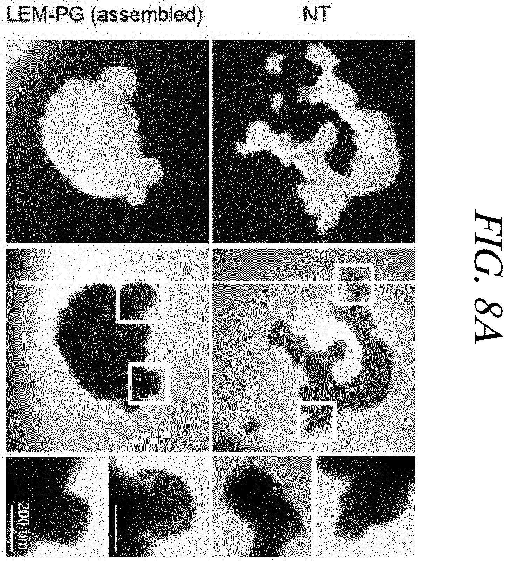

In order to check the assembly efficiency of the prepared LEM-PG and the enhancement of differentiation when assembly of unit liver organoids was induced, it was analyzed by comparison to the group without any treatment with the LEM-PG. In the no treatment (NT) group, 200 hiPSC-derived liver organoids were cultured in a shaker for 7 days without any treatment, and in the LEM-PG group, the LEM-PG (0.5 mg/ml) hydrogel was added to the culture medium at 10% (v/v) to induce assembly. In the NT group, assembly occurred only in part and any aggregation to a single structure did not occur. The liver organoids in which assembly was induced by the LEM-PG formed a large mass and were well organized **(****FIG. 8A****)**.

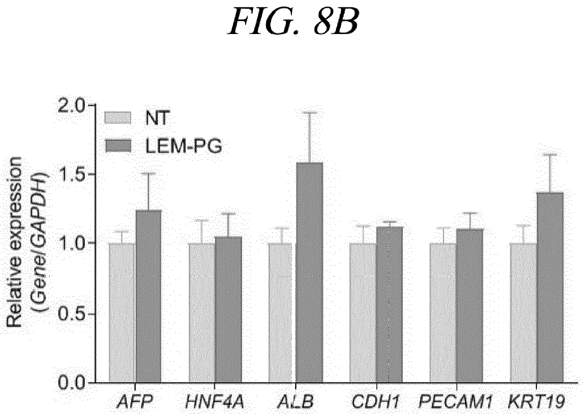

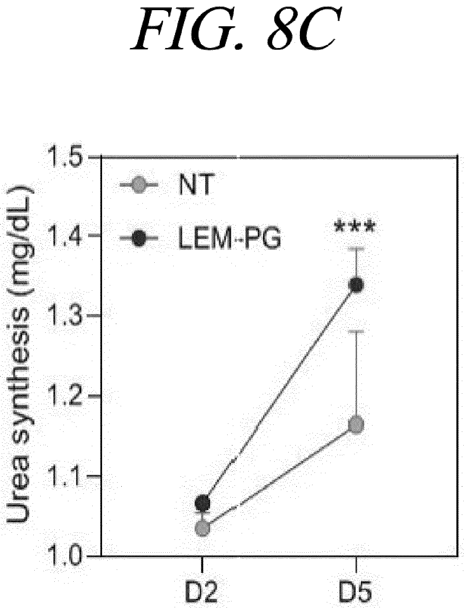

When the mini-liver structure prepared through LEM-PG treatment (LEM-PG group) and the structure without any treatment (NT group) were compared in terms of gene expression, the expression levels of hepatic differentiation markers AFP and HNF4A, a tight junction marker CDH1, a vascular marker PECAM1, and a bile duct marker KRT19 were all high in the group where assembly was induced by the LEM-PG. Also, when the urea synthesis ability was measured on day 2 of culture and day 5 of culture for urea synthesis ability assessment, which is a liver functionality assessment, the structure where liver organoid assembly was induced by the LEM-PG showed significantly high functionality. Accordingly, it was confirmed that the LEM-PG hydrogel not only induces assembly of liver organoids, but also enhances the differentiation and functionality of the organoids due to the effect of the liver tissue-specific LEM **(****FIGS. 8B** and **8C****).**

### Example 4-3. Preparation of ECM-PG hydrogel-based iPSC-derived mini-liver tissue containing liver microenvironment cells

When the LEM-PG hydrogel-based mini-liver structure was prepared to include Kupffer cells and hepatic stellate cells present in the actual liver, the two types of cells were additionally injected and co-cultured during the preparation of unit organoids. Then, the LEM-PG hydrogel was used to induce organoid assembly. In order to prepare a liver tissue analogue containing microenvironment cells at a ratio of hiPSC-derived hepatocytes: vascular endothelial cells: mesenchymal stem cells = 10:7:2 according to a conventional method of preparing a liver organoid, an organoid was prepared by changing a ratio of hiPSC-derived hepatocytes: vascular endothelial cells: mesenchymal stem cells: Kupffer cells: hepatic stellate cells = 10:7:2:2: 1.

Hepatic stellate cells are major cells involved in liver fibrosis and liver cirrhosis by secreting an extracellular matrix (ECM) when activated by various stimuli. Kupffer cells are immune cells that produce antibodies to germs and viruses and inhibit foreign materials entering the body. Both the cells constitute the major microenvironment of liver tissue. Hepatic stellate cells differentiate from iPSC through mesoderm and mesothelial cells. Kupffer cells differentiate from iPSC after production of embryonic bodies (EB) through maturation of macrophage precursors.

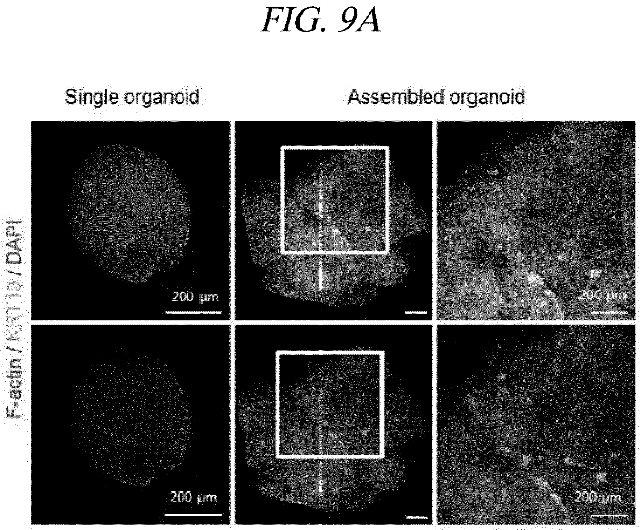

When the mini-liver structure containing microenvironment cells was cultured for 7 days through dynamic culture in a shaker and then immunostained for analysis, the expression of a bile duct cell-related marker was not observed in an unconjugated unit organoid, whereas the expression of a bile duct cell marker KRT19 was observed in parts of the liver structure formed by inducing assembly of 200 organoids using the LEM-PG hydrogel. Accordingly, it was confirmed that when assembly of unit organoids containing microenvironment cells is made efficiently, bile duct cells can also be developed inside the structure **(****FIG. 9A****).** It was confirmed that when liver organoids are assembled and thus increased in size, a niche for maintaining the liver tissue stem cells is formed and bile duct cells can also be formed. Also, it was confirmed that when bile duct cells are formed inside the liver structure, the intrahepatic bile duct, which produces bile, contributes to the survival of hepatocytes, and is essential for artificial liver tissue transplantation, can also be formed, which makes it possible to enhance the liver tissue regeneration effect.

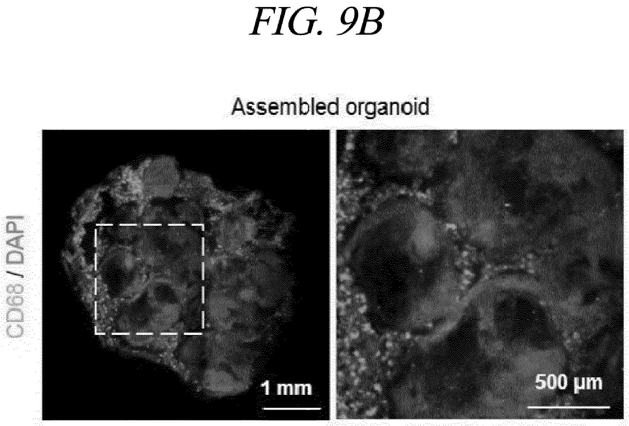

When immunostaining for CD68, a Kupffer cell marker, was performed, Kupffer cells were evenly distributed inside the conjugated liver structure, which confirmed the possibility of preparation of a mini-liver tissue analogue containing microenvironment cells through organoid assembly based on the LEM-PG hydrogel **(****FIG. 9B****).**

### Example 5: Various ECM-PG hydrogel-based organoids and preparation of tissue construct through organoid assembly

### Example 5-1. Preparation of mouse adult stem cell-derived liver tissue construct based on ECM-PG hydrogel

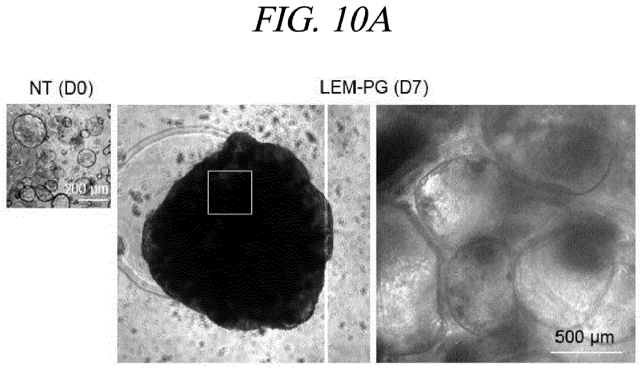

In order to check whether mouse adult stem cell-derived liver organoids can be conjugated to a single structure using the LEM-PG hydrogel, 10% (v/v) LEM-PG hydrogel (final concentration in the culture medium: 0.5 mg/ml) was added to the culture medium in which 200 to 300 mouse adult stem cell-derived liver organoids were cultured. As for liver organoids, mouse bile duct cells containing adult stem cells were extracted and cultured in Matrigel for 7 days. Then, a large amount of organoid was obtained through enzyme treatment and the obtained organoid was used for assembly. When dynamic culture was performed in a shaker for one week, the LEM-PG-treated liver organoids were assembled to a single structure with a size of 4 mm. The internal images also confirmed that the liver organoids were well distributed **(****FIG. 10A****).**

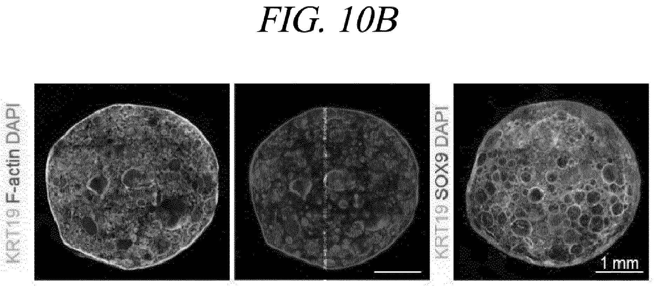

When the expression of the bile duct and hepatic differentiation markers was checked through immunostaining on day 7 of culture, the bile duct and hepatic progenitor-related markers KRT19 and SOX9 were well stained in the organoids inside the prepared liver structure **(****FIG. 10B****).** Accordingly, it was confirmed that the LEM-PG hydrogel can also be used for assembly of adult stem cell-derived liver organoids.

### Example 5-2. Organoid assembly using ECM-PG hydrogel: Pancreatic organoid

In order to check whether the ECM-PG hydrogel optimized for organoid assembly can also be used for assembly of pancreatic organoids, 200 hiPSC-derived pancreatic organoids were coated with a decellularized pancreas extracellular matrix (PEM)-PG hydrogel, which was prepared by modifying the PEM with a gallol group, at a concentration of 10% (v/v) (final concentration in the culture medium of the PEM-PG: 0.5 mg/ml) to induce assembly of the pancreatic organoids.

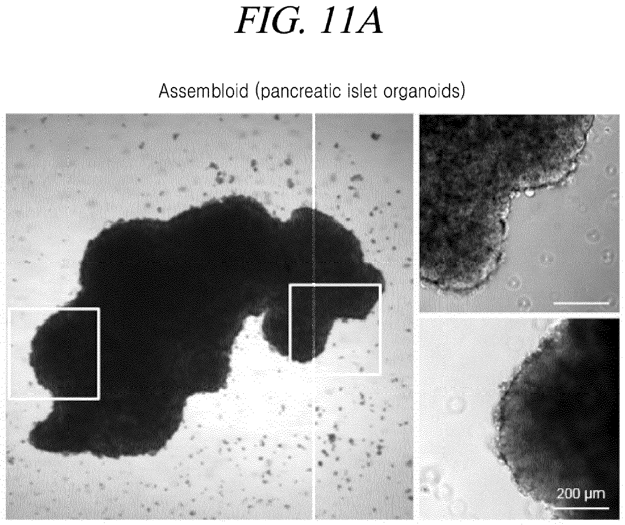

The iPSC-derived pancreatic organoids were prepared through differentiation into pancreatic progenitor cells through definitive endoderm and gut tube endoderm and mixing at a ratio of pancreatic progenitor cells: vascular endothelial cells: mesenchymal stem cells = 10:7:2. When the PEM-PG hydrogel was coated and then cultured in a shaker, the organoids were assembled within 3 days and a mini-pancreatic structure with a size of 1.5 mm to 2 mm was formed **(****FIG. 11A****).** After maturation through additional culture for a total of 7 days to induce differentiation of pancreatic beta cells, immunostaining was performed to analyze the mini-pancreatic structure.

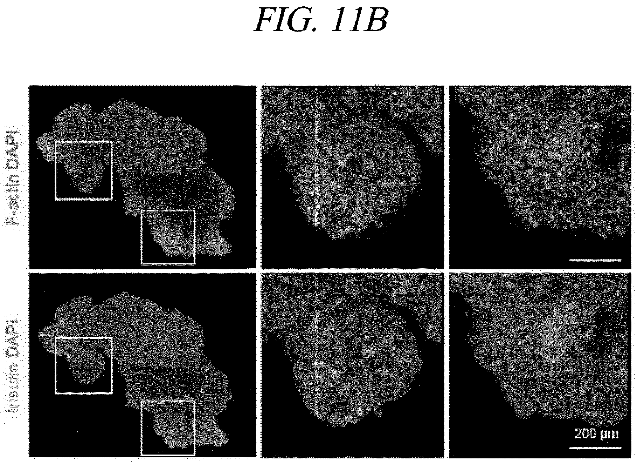

It was confirmed that when the mini-pancreatic structure cultured for 7 days was stained for pancreatic differentiation markers, insulin, a mature pancreatic beta cell differentiation marker, and F-actin, a fibrous filament marker, were well expressed inside the conjugated pancreatic structure **(****FIG. 11B****).** Accordingly, it was verified that the ECM-PG hydrogel can also be used for assembly of mass-produced, iPSC-derived pancreatic organoids.

### Example 5-3. Organoid assembly using ECM-PG hydrogel: Lung organoid

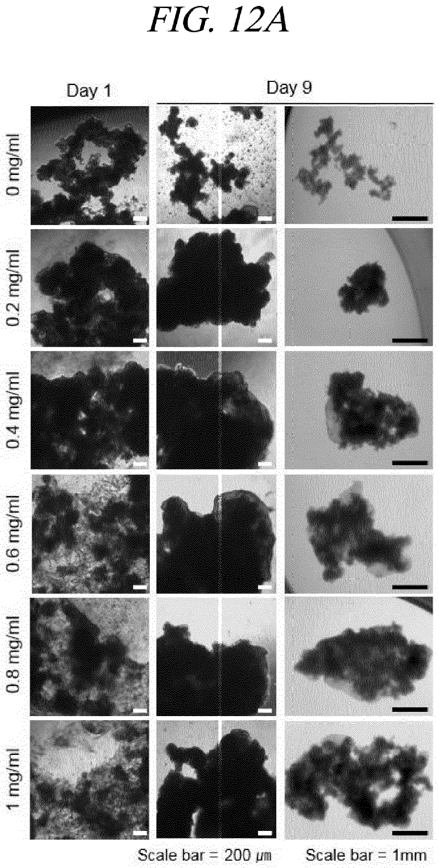

While adult stem cell-derived lung organoids obtained by extracting mouse lung tissue were suspension-cultured, a decellularized lung extracellular matrix (LuEM)-PG hydrogel, which was prepared by modifying the LuEM with a gallol group, was added to the culture medium at various concentrations (final concentration in the culture medium: 0 mg/ml, 0.2 mg/ml, 0.4 mg/ml, 0.6 mg/ml, 0.8 mg/ml and 1 mg/ml). Dynamic culture was carried out in a shaker for 7 days with additional culture for 2 days without the shaker. On day 9 of culture, no assembly of the organoids was observed in the no treatment group, whereas assembly of the lung organoids was induced in the LuEM-PG treatment group **(****FIG. 12A****).** It was confirmed that as the LuEM-PG treatment concentration increased, a larger lung tissue structure was formed **(****FIG. 12A****).**

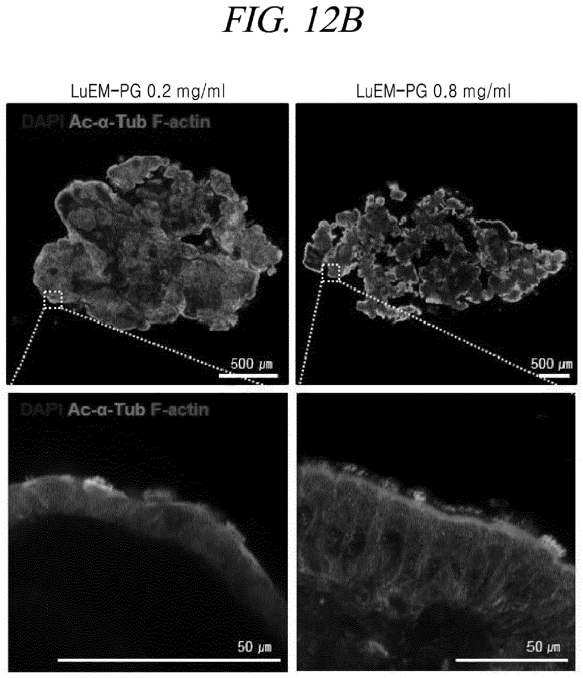

When the organoid groups treated with 0.2 mg/ml and 0.8 mg/ml were selected as a low concentration group and a high concentration group, respectively and the structure was analyzed through immunostaining, the formation of large lung tissue structure through efficient assembly of organoids was observed in the two groups by staining for F-actin marker and the higher expression level of acetylated α-tubulin (Ac-α-Tub), a ciliated cell-related marker, was observed in the 0.8 mg/ml group, *i.e*., the high concentration group **(****FIG. 12B****).** Accordingly, it was confirmed that the LuEM-PG hydrogel can be used to induce assembly of lung organoids and fabricate a lung tissue structure.

### Example 5-4. Organoid assembly using ECM-PG hydrogel: Kidney organoid

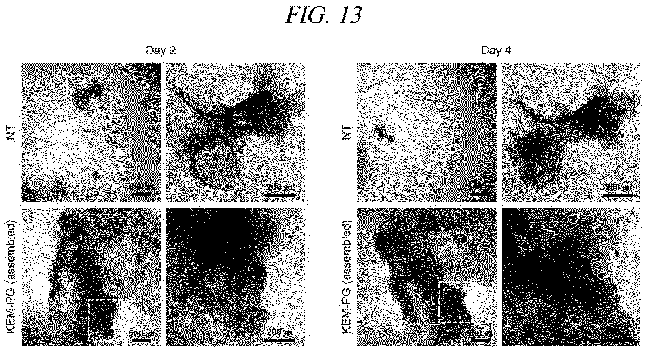

While adult stem cell-derived kidney organoids obtained by extracting mouse kidney tissue were suspension-cultured, a decellularized kidney extracellular matrix (KEM)-PG hydrogel, which was prepared by modifying the KEM with a gallol group, was added to the culture medium at a concentration of 0.5 mg/ml. Dynamic culture was carried out in shakers for 2 days and 4 days, respectively. No assembly of the organoids was observed in the no treatment group, whereas assembly of the kidney organoids was induced in the KEM-PG treatment group **(****FIG. 13****).** Accordingly, it was confirmed that the KEM-PG hydrogel can be used to induce assembly of kidney organoids and fabricate a kidney tissue structure.

### Example 5-5. Organoid assembly using ECM-PG hydrogel: Intestinal organoid

In order to check whether a decellularized intestine extracellular matrix (IEM)-PG hydrogel, which was prepared by modifying the IEM with a gallol group, can also be used for assembly of adult stem cell-derived intestinal organoids to a single structure, 2000 mouse adult stem cell-derived intestinal organoids were cultured in the culture medium where the 10% (v/v) IEM-PG hydrogel (final concentration in the culture medium: 0.4 mg/ml) was added.

As for intestinal organoids, intestinal crypt stem cells were extracted and cultured in Matrigel for 3 days, followed by treatment with a reagent for removing gel to obtain only the organoids. The organoids were used for assembly.

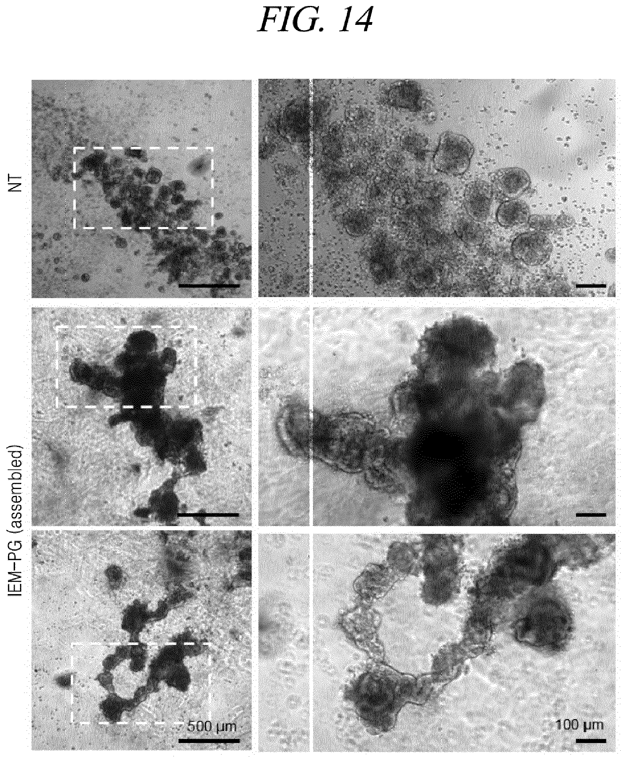

On day 2 of culture with IEM-PG treatment, it was confirmed that the intestinal organoids were assembled to a large structure with a long length into a shape in which the IEM-PG was added **(****FIG. 14****).**

Meanwhile, in the normal culture medium condition (no treatment; NT) where the IEM-PG was not added, the intestinal organoids were not conjugated and were individually arranged. Further, due to the absence of a culture scaffold capable of maintaining/culturing organoids, the morphology was gradually impaired.

Accordingly, it was confirmed that the IEM-PG hydrogel can be used for assembly of adult stem cell-derived intestinal organoids and is helpful in maintaining and growing intestinal organoids even in the absence of a separate culture scaffold.

### Example 5-6. Organoid assembly using ECM-PG hydrogel: Gastricorganoid

A decellularized stomach extracellular matrix (SEM)-PG hydrogel, which was prepared by modifying the SEM with a gallol group, was used to induce assembly of gastric organoids and thus to fabricate large-sized stomach tissues. The gastric organoids were prepared using stem cells extracted from mouse stomach tissue. While the gastric organoids grown to an appropriate size through culture for 4 days were suspension-cultured, the culture medium was treated with the SEM-PG hydrogel at a concentration of 0.5 mg/ml. No treatment (NT) group without any treatment with the SEM-PG hydrogel was compared as a control group.

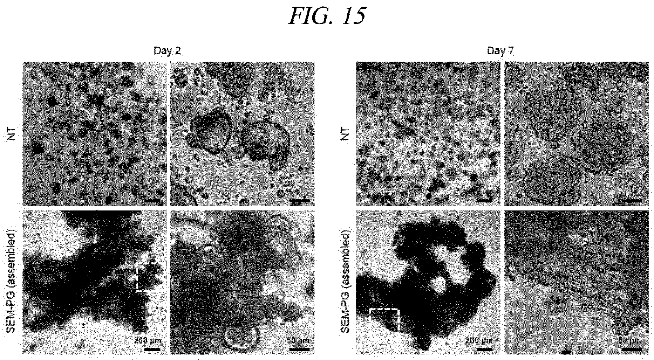

As a result of culture for 2 days, the gastric organoids remained in the form of a single organoid in the NT group, whereas the gastric organoids were conjugated in the assembled group treated with the SEM-PG hydrogel. When the conjugated gastric organoids were cultured for up to 7 days, the gastric organoids were connected to each other to form a large-sized stomach tissue structure. Accordingly, it was confirmed that the SEM-PG hydrogel can be used to induce assembly of gastric organoids and fabricate a stomach tissue structure **(****FIG. 15****).**

### Example 5-7. Organoid assembly using ECM-PG hydrogel: Esophageal organoid

While esophageal organoids prepared by extracting stem cells from mouse esophageal tissue were suspension-cultured, a decellularized esophagus extracellular matrix (EEM)-PG hydrogel, which was prepared by modifying the EEM with a gallol group, was added to the culture medium to induce assembly of the esophageal organoids.

About 2000 to 2500 esophageal organoids per group were used for assembly, and the EEM-PG hydrogel was added to the culture medium to reach the final concentration of 0.2 mg/ml and 0.4 mg/ml.

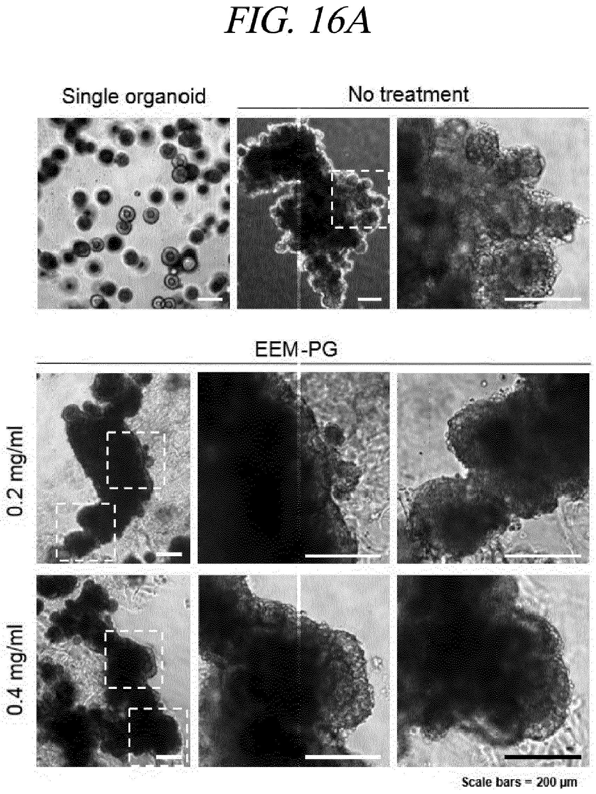

As a result of culture for 5 days in a shaker, assembly and smooth connection of the organoids were not properly made in the no treatment group (NT), whereas assembly of the organoids was induced to form a large-sized esophageal structure in the EEM-PG treatment group **(****FIG. 16A****).**

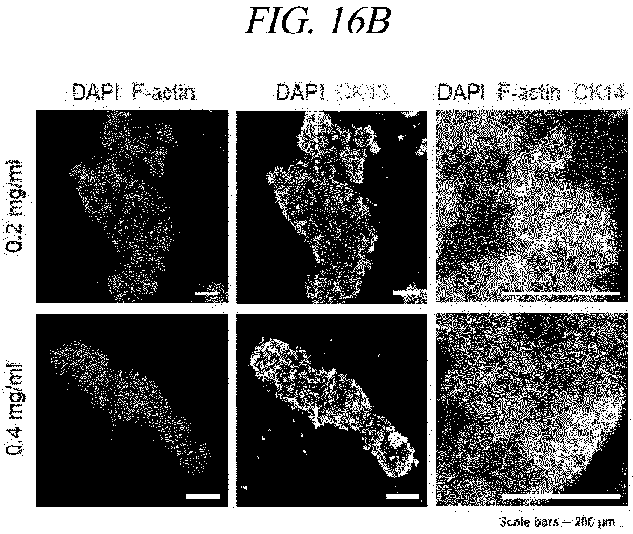

As a result of immunostaining of the esophageal structure cultured for 5 days, it was confirmed that assembly of the organoids was well made at both concentrations of 0.2 mg/ml and 0.4 mg/ml through staining for F-actin, and both cytokeratin 13 (CK13) expressed in the suprabasal layer of the esophagus and cytokeratin 14 (CK14) expressed in the basal layer were well expressed in the structure **(****FIG. 16B****).**

Accordingly, it was confirmed that the EEM-PG hydrogel can be used to induce assembly of esophageal organoids and fabricate an esophageal structure.

### Example 6: Confirmation of applicability of ECM-PG hydrogel

### Example 6-1. Tissue adhesion test of ECM-PG patch

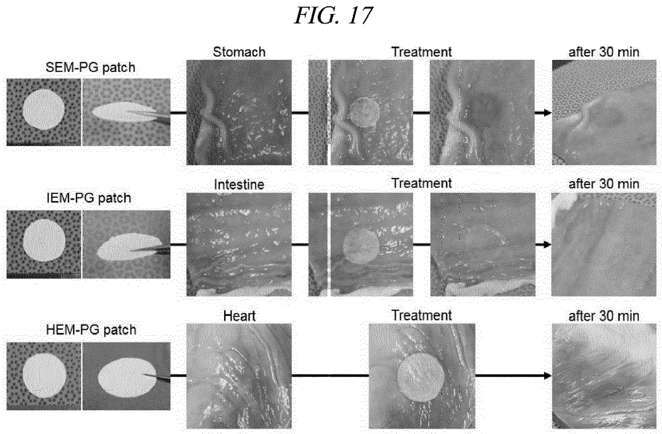

Each of a stomach extracellular matrix (SEM) from decellularized stomach tissue, an intestine extracellular matrix (IEM) from decellularized intestine tissue, and a heart extracellular matrix (HEM) from decellularized heart tissue was modified with a gallol group through an EDC/NHS reaction, and each of the synthesized tissue ECM-PG derivatives (SEM-PG, IEM-PG and HEM-PG) was dissolved in a solution (final concentration of 1% (w/v) in the PBS buffer), poured into a circular mold, and freeze-dried to prepare ECM-PG patches. As can be seen from **FIG. 17****,** each ECM-PG patch adheres well to the tissue surface while crosslinking occurs due to natural oxidation of the gallol group over time without the addition of a crosslinking agent.

In this way, the tissue-specific extracellular matrix can be effectively delivered into the tissue, and when the matrix is loaded with a drug and delivered, the therapeutic effect by the drug and the tissue regeneration effect by the tissue-specific extracellular matrix can be simultaneously induced. Therefore, it can be applied as a drug delivery system for tissue regeneration of injured tissue.

### Example 6-2. Mass transplantation of iPSC-derived liver organoid using ECM-PG patch

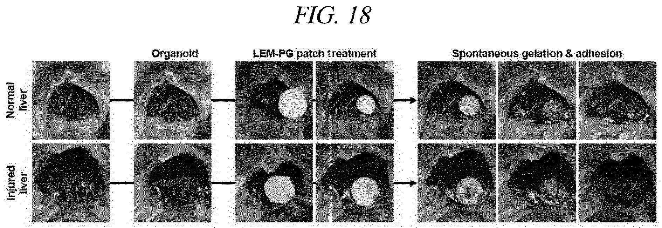

When 100 iPSC-derived liver organoids were collected and placed on the mouse liver tissue (normal liver; top) and then the ECM-PG patch (herein, the LEM-PG patch) was placed thereon to perform organoid mass transplantation, hydrogel formation and adhesion by natural oxidation of the gallol group was induced after about 30 minutes even without the addition of a separate additive (oxidizing agent) for crosslinking, as can be seen from **FIG. 18****.** Thus, it was confirmed that the organoids can be easily transplanted into living tissue.

In particular, it was confirmed that a large amount of organoids was completely transplanted using the ECM-PG patch even in the injured area (injured liver; bottom) where bleeding occurred due to tissue injury. Therefore, it was confirmed that non-invasive mass transplantation of organoids into the injured area can be made and the ECM-PG patch technology developed in the present disclosure can be applied to tissue regeneration treatment.

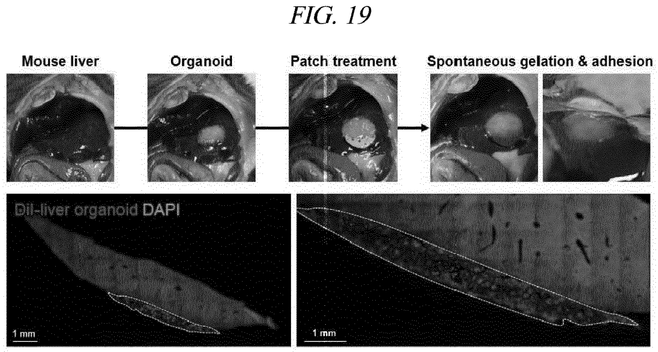

Further, 200 hiPSC-derived liver organoids were collected and placed on the injured liver tissue where bleeding occurred due to tissue injury, and then the prepared ECM-PG patch (LEM-PG patch) was placed thereon to perform transplantation, which confirmed that organoid mass transplantation can be effectively performed into the mouse liver tissue. As a result, as shown in **FIG. 19****,** even when tissue sections were fabricated and observed 24 hours after transplantation, liver organoids labeled with red fluorescence (Dil) were evenly transplanted to the injured area with a size of 5 mm and stably located, which shows the ECM-PG patch technology developed in the present disclosure can also be applied to non-invasive mass transplantation of a large amount of organoid with a large size.

### Example 6-3. Transplantation of iPSC-derived mini-liver tissue prepared using ECM-PG hydrogel into liver perforation model

In order to check the transplantability of the prepared mini-liver structure and the tissue regeneration, a liver tissue structure with a size of 4 mm to 5 mm was first fabricated by inducing assembly of 200 hiPSC-derived unit liver organoids and then transplanted into a liver perforation model by using the LEM-PG patch. It was confirmed that when assembly was induced by using the LEM-PG hydrogel, the unit organoids were conjugated to a large structure with a size of 4 mm to 5 mm within 3 days **(****FIG. 20A****).**

The liver structure was transplanted by inducing a 6-mm perforation in the mouse liver tissue with a biopsy punch, placing the liver tissue structure (assembloid) assembled by the LEM-PG hydrogel onto the liver tissue injured by perforation, and covering the area where the structure was located with the LEM-PG patch **(****FIG. 20B****).** A week after the assembloid was transplanted around the perforation site, immunostaining and histological analysis were performed to check whether the transplanted liver tissue is engrafted and functioned well. H&E staining confirmed that the assembloid transplanted into the perforation site was well engrafted and the LEM-PG patch coated thereon was also well maintained **(****FIG. 20C****).** Staining for ALB, a hepatic differentiation marker, confirmed that the transplanted assembloid well expressed the ALB differentiation marker even a week later **(****FIG. 20D****).**

Accordingly, it was verified that mini-liver tissues can be produced through assembly of unit liver organoids with the LEM-PG hydrogel and the tissues can be transplanted into a liver tissue injury model by using the LEM-PG patch.

### Example 6-4. Verification of effect of transplantation of iPSC-derived mini-liver tissue structure prepared based on ECM-PG hydrogel into partial liver resection model

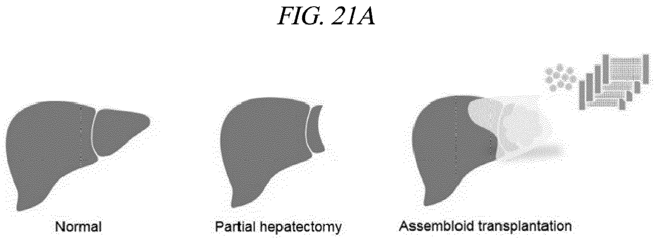

In order to check the transplantability of the prepared mini-liver tissue analogue and the tissue regeneration into a liver resection model, hiPSC-derived unit liver organoids were mass-produced and assembly of the organoids was induced by using the LEM-PG hydrogel (final concentration of the LEM-PG added into the culture medium: 0.5 mg/ml) to prepare an assembloid with a size of 6 mm to 8 mm **(****FIG. 21A****).**

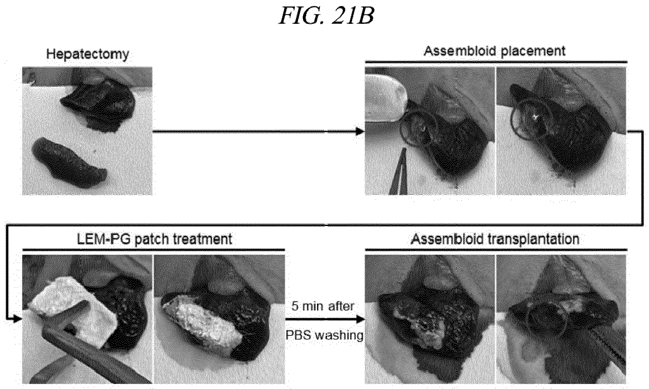

A partial liver resection model (50% partial hepatectomy) was prepared by resecting 50% of the left lateral lobe of the mouse liver tissue, and a mini-liver tissue was placed on the injured liver tissue, and the LEM-PG patch (prepared by solutionizing an LEM-PG derivative at a concentration of 1% (w/v) and pouring the solution into a mold, followed by freeze-drying) was covered thereto to perform transplantation **(****FIG. 21B****).** In order to prepare a mini-liver tissue that can be applied to a mouse liver resection model, assembly of 400 unit liver organoids was induced.

After 50% of the left lateral lobe of the mouse liver tissue was resected, followed by hemostasis, the mini-liver tissue was transplanted by using the LEM-PG patch. As a result, the structure was effectively engrafted on the surface of the injured liver tissue **(****FIG. 21B****).**

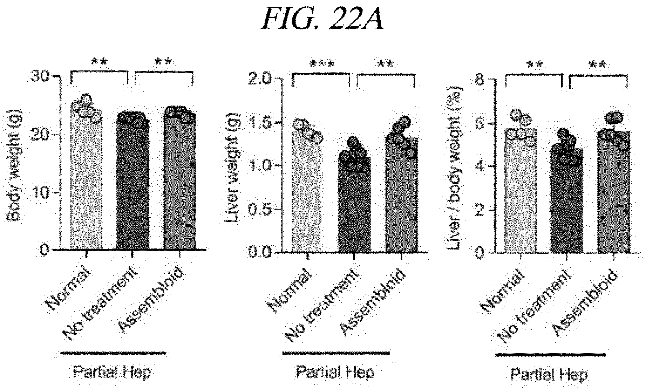

Further, when the mouse body weight and liver weight of each group were measured 2 weeks after transplantation, the recovery rate was slow and the body weight and liver weight decreased compared to a normal mouse in the no treatment group in which partial liver resection was induced without any treatment, whereas the body weight and liver weight were recovered to a certain extent in the partial liver resection group in which the assembloid was transplanted. Furthermore, the ratio of liver weight to body weight was low in the no treatment group compared to the normal mouse, whereas the ratio of liver weight to body weight was also recovered to a certain extent in the assembloid-transplanted group **(****FIG. 22A****).**

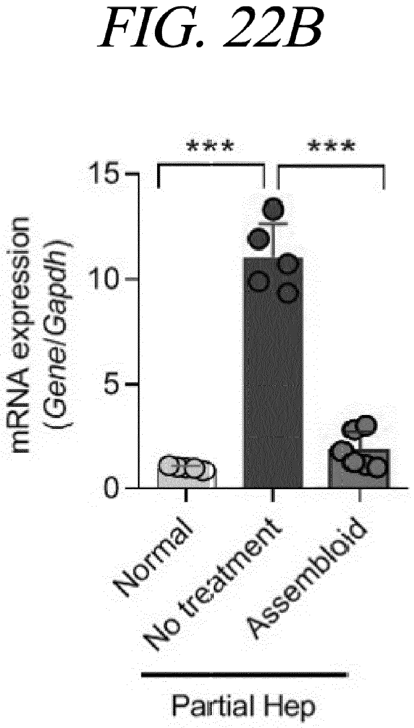

In order to compare the expression of fibrosis markers in each group at week 2 of transplantation, mRNA was extracted from the tissue and quantitative PCR (qPCR) analysis was performed. As a result, the expression level of Col1a1 in the no treatment group was increased by about 10 times compared to the normal liver tissue, whereas the expression level of Col1a1 in the assembloid-transplanted group was greatly reduced to a significant level since tissue regeneration was induced in the assembloid-transplanted group **(****FIG. 22B****).**

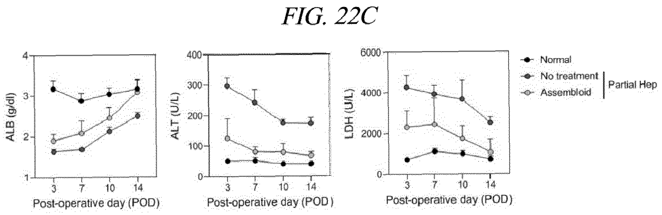

Blood was collected on days 3, 7, 10 and 14 after transplantation of the assembloid to perform blood analysis for ALB and LDH, liver function indicators and major liver toxicity indicators. While there was no significant change in ALB, ALT and LDH levels in the normal mouse group, the ALT and LDH levels were very high and the amount of ALB was low in the no treatment group in which partial liver resection was induced. The no treatment group showed recovery to a certain extent for 2 weeks, but still had a low blood ALB level and high liver toxicity (ALT and LDH) levels. However, in the group (assembloid) in which the assembloid was transplanted by using the LEM-PG patch, the blood ALB level was rapidly recovered and the liver toxicity level was also significantly reduced, which confirmed that the liver injury was rapidly recovered **(****FIG. 22C****).** Therefore, it was confirmed that the liver functionality inhibited by partial liver resection was recovered to a certain extent and the liver injury was reduced by the transplanted mini-liver tissue.

Accordingly, it was confirmed that the developed LEM-PG can be used as a hydrogel for organoid assembly to produce liver tissue and applied in the form of a patch to efficiently transplant the tissue.

Further, at week 2 of transplantation of the mini-liver assembloid produced by using the LEM-PG hydrogel into a partial hepatectomy mouse model by using the LEM-PG patch, H&E histological analysis and immunostaining were performed to check the engrafted mini-liver tissue.

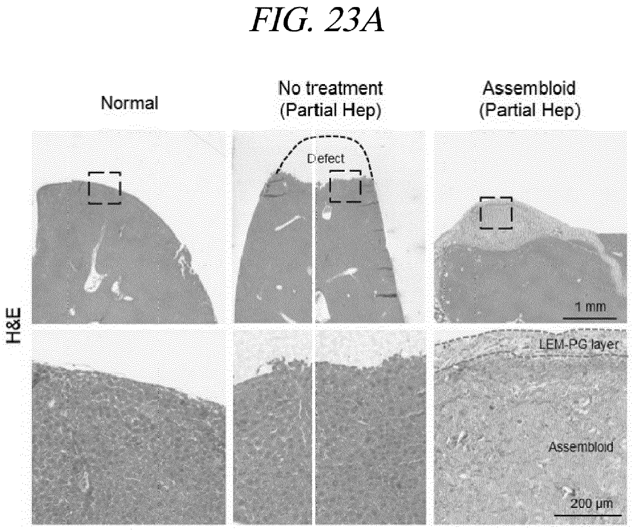

In the no treatment group in which 50% of the left lateral lobe of the mouse liver tissue was resected without any treatment, the injured tissue was not well regenerated compared to the normal liver tissue. In the group in which the assembloid was transplanted by using the LEM-PG patch, the injured area was effectively filled by the transplanted assembloid and the structure was well engrafted on the surface of the injured liver tissue for 2 weeks **(****FIG. 23A****).**

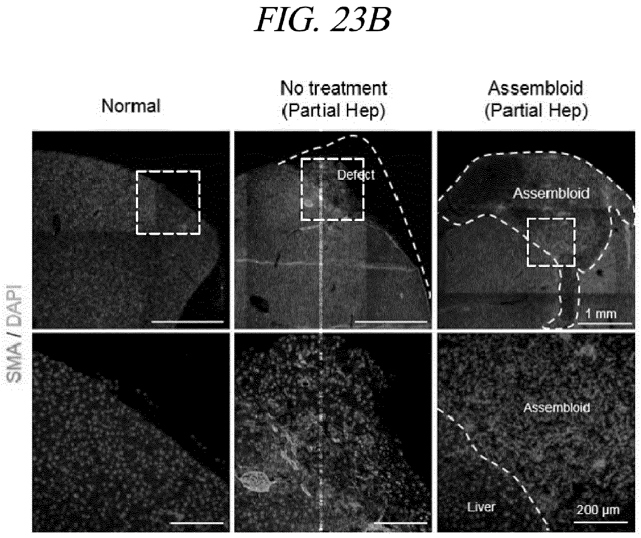

When immunostaining for SMA, a fibrosis marker, was performed for each group, the expression level of the SMA marker was high in the no treatment group because fibrosis actively occurred in the injured tissue area (defect), whereas the expression of the SMA was hardly observed in the assembloid-transplanted group due to the tissue regeneration effect of the transplanted assembloid **(****FIG. 23B****).**

Accordingly, it was confirmed that the developed LEM-PG can be used as a hydrogel for organoid assembly to produce liver tissue and applied in the form of a patch to efficiently transplant the tissue.

The present disclosure has been described with reference to the preferred exemplary embodiments thereof. Accordingly, the above-described exemplary embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. A composition for hydrogel, comprising:
a phenol derivative-modified, tissue-derived extracellular matrix.

2. The composition for hydrogel of Claim 1,
wherein the phenol derivative is derived from at least one of a catechol group ( 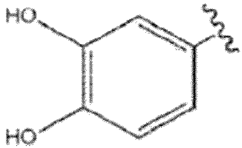 ) and a gallol group ( 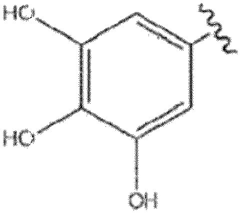 ).

3. The composition for hydrogel of Claim 1,
wherein the tissue-derived extracellular matrix is any one of a liver tissue-derived extracellular matrix, a lung tissue-derived extracellular matrix, a stomach tissue-derived extracellular matrix, an intestine tissue-derived extracellular matrix, a kidney tissue-derived extracellular matrix, a heart tissue-derived extracellular matrix, an esophageal tissue-derived extracellular matrix, a brain tissue-derived extracellular matrix, a spinal cord tissue-derived extracellular matrix, a pancreatic tissue-derived extracellular matrix, a uterine tissue-derived extracellular matrix, and an adipose tissue-derived extracellular matrix.

4. The composition for hydrogel of Claim 1,
wherein the weight ratio of the phenol derivative and the tissue-derived extracellular matrix is 1:10 to 10:1.

5. A hydrogel prepared by crosslinking the composition of Claim 1.

6. The hydrogel of Claim 5,
wherein the composition has a concentration of 0.01%(w/v) to 3% (w/v).

7. The hydrogel of Claim 5,
wherein the crosslinking is oxidative crosslinking.

8. The hydrogel of Claim 7,
wherein the oxidative crosslinking is performed through at least one of a reaction by treatment with an oxidizing agent, a reaction under basic conditions, and natural oxidation.

9. The hydrogel of Claim 5,
wherein the hydrogel has adhesiveness.

10. The hydrogel of Claim 9,
wherein the adhesiveness is caused by at least one of a nucleophilic covalent bond, a hydrogen bond, a hydrophobic interaction, and a pi-pi interaction with the modified phenol derivative.

11. The hydrogel of Claim 5,
wherein the hydrogel is biodegradable.

12. A tissue adhesive composition, comprising:
the hydrogel of Claim 5.

13. A composition for cell culture, comprising:
the hydrogel of Claim 5.

14. A tissue structure to which the cell-coated composition of Claim 13 adheres.

15. A composition for drug delivery, comprising:
the hydrogel of Claim 5.

16. A composition for cell transplantation, comprising:
the hydrogel of Claim 5.

17. A method of preparing a hydrogel, comprising:
a process (a) of modifying a tissue-derived extracellular matrix with a phenol derivative; and
a process (b) of crosslinking the modified, tissue-derived extracellular matrix to form a hydrogel.

18. The method of Claim 17,
wherein the crosslinking in the process (b) is oxidative crosslinking.

19. The method of Claim 18,
wherein the oxidative crosslinking is performed through at least one of a reaction by treatment with an oxidizing agent, a reaction under basic conditions, and natural oxidation.

20. The method of Claim 19,
wherein the reaction by treatment with an oxidizing agent or the reaction under basic conditions is carried out by treating a substituted, tissue-derived extracellular matrix with at least one of NaOH, NalO_{α}, Na₂S₂O₈ and Fe³⁺.

## Patentansprüche

1. Zusammensetzung für Hydrogel, umfassend:
eine Phenolderivat-modifizierte, aus Gewebe stammende extrazelluläre Matrix.

2. Zusammensetzung für Hydrogel nach Anspruch 1,
wobei das Phenolderivat von mindestens einer von einer Brenzcatechingruppe ( 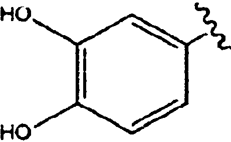 ) und einer Gallolgruppe ( 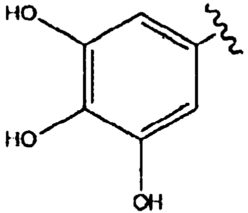 ) abgeleitet ist.

3. Zusammensetzung für Hydrogel nach Anspruch 1,
wobei die aus Gewebe stammende extrazelluläre Matrix eine von einer aus Lebergewebe stammenden extrazellulären Matrix, einer aus Lungengewebe stammenden extrazellulären Matrix, einer aus Magengewebe stammenden extrazellulären Matrix, einer aus Darmgewebe stammenden extrazellulären Matrix, einer aus Nierengewebe stammenden extrazellulären Matrix, einer aus Herzgewebe stammenden extrazellulären Matrix, einer aus Speiseröhrengewebe stammenden extrazellulären Matrix, einer aus Hirngewebe stammenden extrazellulären Matrix, einer aus Rückenmarksgewebe stammenden extrazellulären Matrix, einer aus Bauchspeicheldrüsengewebe stammenden extrazellulären Matrix, einer aus Gebärmuttergewebe stammenden extrazellulären Matrix und einer aus Fettgewebe stammenden extrazellulären Matrix ist.

4. Zusammensetzung für Hydrogel nach Anspruch 1,
wobei das Gewichtsverhältnis des Phenolderivats und der aus Gewebe stammenden extrazellulären Matrix 1:10 bis 10:1 beträgt.

5. Hydrogel, hergestellt durch Vernetzen der Zusammensetzung nach Anspruch 1.

6. Hydrogel nach Anspruch 5,
wobei die Zusammensetzung eine Konzentration von 0,01% (w/v) bis 3% (w/v) aufweist.

7. Hydrogel nach Anspruch 5,
wobei das Vernetzen oxidatives Vernetzen ist.

8. Hydrogel nach Anspruch 7,
wobei das oxidative Vernetzen durch mindestens eine von einer Reaktion durch Behandlung mit einem Oxidationsmittel, einer Reaktion unter basischen Bedingungen und natürlicher Oxidation durchgeführt ist.

9. Hydrogel nach Anspruch 5,
wobei das Hydrogel Haftvermögen aufweist.

10. Hydrogel nach Anspruch 9,
wobei das Haftvermögen durch mindestens eine von einer nukleophilen kovalenten Bindung, einer Wasserstoffbrückenbindung, einer hydrophoben Wechselwirkung und einer Pi-Pi-Wechselwirkung mit dem modifizierten Phenolderivat verursacht ist.

11. Hydrogel nach Anspruch 5,
wobei das Hydrogel bioabbaubar ist.

12. Gewebeklebstoffzusammensetzung, umfassend:
das Hydrogel nach Anspruch 5.

13. Zusammensetzung für Zellkultur, umfassend:
das Hydrogel nach Anspruch 5.

14. Gewebestruktur, an der die zellbeschichtete Zusammensetzung nach Anspruch 13 haftet.

15. Zusammensetzung für Arzneistoffzufuhr, umfassend:
das Hydrogel nach Anspruch 5.

16. Zusammensetzung für Zelltransplantation, umfassend:
das Hydrogel nach Anspruch 5.

17. Verfahren zur Herstellung eines Hydrogels, umfassend:
einen Prozess (a) des Modifizierens einer aus Gewebe stammenden extrazellulären Matrix mit einem Phenolderivat; und
einen Prozess (b) des Vernetzens der modifizierten, aus Gewebe stammenden extrazellulären Matrix, um ein Hydrogel zu bilden.

18. Verfahren nach Anspruch 17,
wobei das Vernetzen im Prozess (b) oxidatives Vernetzen ist.

19. Verfahren nach Anspruch 18,
wobei das oxidative Vernetzen durch mindestens eine von einer Reaktion durch Behandlung mit einem Oxidationsmittel, einer Reaktion unter basischen Bedingungen und natürlicher Oxidation durchgeführt wird.

20. Verfahren nach Anspruch 19,
wobei die Reaktion durch Behandlung mit einem Oxidationsmittel oder die Reaktion unter basischen Bedingungen durch Behandeln einer substituierten, aus Gewebe stammenden extrazellulären Matrix mit mindestens einem von NaOH, NaIO₄, Na₂S₂O₈ und Fe³⁺ ausgeführt wird.

## Revendications

1. Composition pour hydrogel, comprenant :
un dérivé de matrice extracellulaire dérivée d'un tissu, modifié par un dérivé de phénol.

2. Composition pour hydrogel selon la revendication 1,
dans laquelle le dérivé de phénol est dérivé d'au moins un groupement parmi un groupement catéchol ( 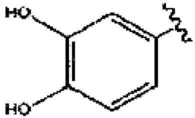 ) et un groupement gallol ( 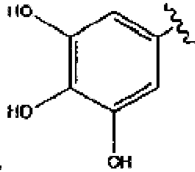 ).

3. Composition pour hydrogel selon la revendication 1,
dans laquelle la matrice extracellulaire dérivée d'un tissu est l'une quelconque parmi une matrice extracellulaire dérivée d'un tissu du foie, une matrice extracellulaire dérivée d'un tissu d'un poumon, une matrice extracellulaire dérivée de tissu de l'estomac, une matrice extracellulaire dérivée d'un tissu de l'intestin, une matrice extracellulaire dérivée d'un tissu d'un rein, une matrice extracellulaire dérivée d'un tissu du cœur, une matrice extracellulaire dérivée d'un tissu de l'œsophage, une matrice extracellulaire dérivée d'un tissu du cerveau, une matrice extracellulaire dérivée d'un tissu de la moelle épinière, une matrice extracellulaire dérivée d'un tissu pancréatique, une matrice extracellulaire dérivée d'un tissu utérin, et une matrice extracellulaire dérivée d'un tissu adipeux.

4. Composition pour hydrogel selon la revendication 1,
dans laquelle le ratio en poids du dérivé de phénol et de la matrice extracellulaire dérivée d'un tissu est de 1 : 10 à 10 : 1.

5. Hydrogel préparé par réticulation de la composition selon la revendication 1.

6. Hydrogel selon la revendication 5,
dans lequel la composition a une concentration de 0,01 % (p/v) à 3 % (p/v).

7. Hydrogel selon la revendication 5,
dans lequel la réticulation est une réticulation oxydative.

8. Hydrogel selon la revendication 7,
dans lequel la réticulation oxydative est effectuée par au moins une réaction parmi une réaction de traitement avec un agent d'oxydation, une réaction dans des conditions basiques, et une oxydation naturelle.

9. Hydrogel selon la revendication 5,
l'hydrogel possédant de l'adhésivité.

10. Hydrogel selon la revendication 9,
dans lequel l'adhésivité est causée par au moins un élément parmi une liaison covalente nucléophile, une liaison hydrogène, une interaction hydrophobe, et une interaction pi-pi avec le dérivé de phénol modifié.

11. Hydrogel selon la revendication 5,
l'hydrogel étant biodégradable.

12. Composition de tissu adhésive comprenant :
l'hydrogel selon la revendication 5.

13. Composition pour culture cellulaire comprenant :
l'hydrogel selon la revendication 5.

14. Structure de tissu à laquelle la composition cellulaire revêtue selon la revendication 13 adhère.

15. Composition pour l'administration d'un médicament comprenant :
l'hydrogel selon la revendication 5.

16. Composition pour transplantation cellulaire comprenant :
l'hydrogel selon la revendication 5.

17. Procédé de préparation d'un hydrogel comprenant :
un processus (a) de modification d'une matrice extracellulaire dérivée d'un tissu avec un dérivé de phénol ; et
un processus (b) de réticulation de la matrice extracellulaire dérivée d'un tissu modifiée pour former un hydrogel.

18. Procédé selon la revendication 17,
dans lequel la réticulation dans le processus (b) est une réticulation oxydative.

19. Procédé selon la revendication 18,
dans lequel la réticulation oxydative est effectuée par au moins une réaction parmi une réaction de traitement avec un agent d'oxydation, une réaction dans des conditions basiques et une oxydation naturelle.

20. Procédé selon la revendication 19,
dans lequel la réaction de traitement avec un agent d'oxydation ou la réaction dans des conditions basiques est effectuée en traitant une matrice extracellulaire dérivée d'un tissu substituée avec au moins un composé parmi NaOH, NaIO₄, Na₂S₂O₈ et Fe³⁺.
